# EUROPEAN PATENT SPECIFICATION

(11) **EP 2 079 851 B1**
(45) Date of publication and mention of the grant of the patent: **07.01.2015**
(21) Application number: 07864115.6
(22) Date of filing: 08.11.2007
(51) Int. Cl.: C12Q 1/68, G01N 33/574

(54) **METHOD USING SPINK1 AS A PROSTATE CANCER MARKER**
VERFAHREN DAS SPINK1 ALS MARKER FÜR PROSTATAKREBS VERWENDET
PROCÉDÉ UTILISANT SPINK1 EN TANT QUE MARQUEUR DU CANCER DE LA PROSTATE

(30) Priority: 08.11.2006 US 857758 P; 14.03.2007 US 906876 P
(43) Date of publication of application: 22.07.2009
(73) Proprietor: The Regents of The University of Michigan, Ann Arbor, MI 48109-2590 (US)
(72) Inventor: CHINNAIYAN, Arul M., Plymounth, MI 48170 (US); TOMLINS, Scott A., Ann Arbor, MI 48105 (US); RHODES, Daniel R., Ann Arbor, MI 48105 (US); MEHRA, Rohit, Ann Arbor, MI 48105 (US)
(74) Representative: van der Hoff, Hilary Mary
(86) International application number: PCT/US2007/084090
(87) International publication number: WO 2008/058239

(56) References cited:
- BJARTELL A ET AL: "TUMOR-ASSOCIATED TRYPSIN INHIBITOR (TATI, PSTI, SPINK1) EXPRESSION IN PROSTATE CANCER IS RELATED TO TUMOR GRADE" EUROPEAN UROLOGY SUPPLEMENTS, XX, XX LNKD- DOI:10.1016/S1569-9056(06)61267-0, vol. 5, no. 14, 1 September 2006 (2006-09-01), page 791, XP025122254 ISSN: 1569-9056 [retrieved on 2006-09-01]
- PAJU ET AL: "Increased Expression of Tumor-Associated Trypsin Inhibitor, TATI, in Prostate Cancer and in Androgen-Independent 22Rv1 Cells" EUROPEAN UROLOGY, ELSEVIER BV, NL LNKD- DOI:10.1016/J.EURURO.2007.01.096, vol. 52, no. 6, 5 February 2007 (2007-02-05), pages 1670-1681, XP022356369 ISSN: 0302-2838
- SINGH JAS ET AL: "RNA interference mediated silencing of SPINK reduces invasion and proliferation of prostate cancer cells" PROCEEDINGS OF THE ANNUAL MEETING OF THE AMERICAN ASSOCIATION FOR CANCER RESEARCH, AMERICAN ASSOCIATION FOR CANCER RESEARCH, US, vol. 47, 1 April 2006 (2006-04-01), page 823, XP009135622 ISSN: 0197-016X
- LAPOINTE J ET AL: "Gene expression profiling identifies clinically relevant subtypes ofprostate cancer" PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.0304146101, vol. 101, no. 3, 1 January 2004 (2004-01-01), pages 811-816, XP002497761 ISSN: 0027-8424
- FRADET Y ET AL: "APTIMA(R) PCA3 MOLECULAR URINE TEST: DEVELOPMENT OF A METHOD TO AID IN THE DIAGNOSIS OF PROSTATE CANCER" EUROPEAN UROLOGY SUPPLEMENTS, XX, XX LNKD- DOI:10.1016/S1569-9056(06)61015-4, vol. 5, no. 2, 1 April 2006 (2006-04-01), page 275, XP025121930 ISSN: 1569-9056 [retrieved on 2006-04-01]
- LI SHIJUN ET AL: "Application of genomic technologies to human prostate cancer" OMICS A JOURNAL OF INTEGRATIVE BIOLOGY, vol. 10, no. 3, September 2006 (2006-09), pages 261-275, XP002590178 ISSN: 1536-2310
- BHARATHI LAXMAN ET AL: "Noninvasive Detection of TMPRSS2:ERG Fusion Transcripts in the Urine of Men with Prostate Cancer" NEOPLASIA, NEOPLASIA PRESS, ANN ARBOR, MI, US LNKD- DOI:10.1593/NEO.06625, vol. 8, no. 10, 1 October 2006 (2006-10-01), pages 885-888, XP007906803 ISSN: 1522-8002
- LAXMAN BHARATHI ET AL: "A first-generation multiplex biomarker analysis of urine for the early detection of prostate cancer" CANCER RESEARCH, vol. 68, no. 3, February 2008 (2008-02), pages 645-649, XP002590179 ISSN: 0008-5472
- SINGH J. ET AL.: 'Annotation of androgen dependence to human prostate cancer associated genes by microarray analysis of mouse prostate' CANCER LETTERS vol. 237, 2006, pages 298 - 304, XP005463612
- 'Affymetric NETAFFX details for MG-U7AV2', [Online] 18 August 2008, XP008109542 Retrieved from the Internet: <URL:http://www.Affymetrix.com>

## Description

### FIELD OF THE INVENTION

Kits and methods for cancer research and diagnosis, including but not limited to, cancer markers are provided. In particular, SPINK1 and other markers for prostate cancer are provided.

### BACKGROUND OF THE INVENTION

Prostate cancer is the most common nondermatologic cancer and the second most common cause of cancer-related deaths in American men. The number of prostate cancers recorded in cancer registries in the United States and the United Kingdom has increased markedly in the past 15 years. This change predominantly represents an increase in the number of cancers diagnosed rather than a real increase in the number of cancers in the population. In 2006, 234,460 new cases and 27,350 deaths were estimated to occur. It was determined that approximately 91 % of these new cases would be diagnosed at local or regional stages.

Prostate cancer (PCa) is typically diagnosed with a digital rectal exam and/or prostate specific antigen (PSA) screening. An elevated serum PSA level can indicate the presence of PCa. PSA is used as a marker for prostate cancer because it is secreted only by prostate cells. A healthy prostate will produce a stable amount-typically below 4 nanograms per milliliter, or a PSA reading of "4" or less -- whereas cancer cells produce escalating amounts that correspond with the severity of the cancer. A level between 4 and 10 may raise a doctor's suspicion that a patient has prostate cancer, while amounts above 50 may show that the tumor has spread elsewhere in the body.

When PSA or digital tests indicate a strong likelihood that cancer is present, a transrectal ultrasound (TRUS) is used to map the prostate and show any suspicious areas. Biopsies of various sectors of the prostate are used to determine if prostate cancer is present. Treatment options depend on the stage of the cancer. Men with a 10-year life expectancy or less who have a low Gleason number and whose tumor has not spread beyond the prostate are often treated with watchful waiting (no treatment). Treatment options for more aggressive cancers include surgical treatments, such as radical prostatectomy (RP) in which the prostate is completely removed (with or without nerve sparing techniques), and radiation, applied through an external beam that directs the dose to the prostate from outside the body or via low-dose radioactive seeds that are implanted within the prostate to kill cancer cells locally. Anti-androgen hormone therapy is also used, alone or in conjunction with surgery or radiation. Hormone therapy uses luteinizing hormone-releasing hormones (LH-RH) analogs, which block the pituitary from producing hormones that stimulate testosterone production. Patients must have injections of LH-RH analogs for the rest of their lives.

While surgical and hormonal treatments are often effective for localized PCa, advanced disease remains essentially incurable. Androgen ablation is the most common therapy for advanced PCa, leading to massive apoptosis of androgen-dependent malignant cells and temporary tumor regression. In most cases, however, the tumor reemerges with a vengeance and can proliferate independent of androgen signals.

The advent of PSA screening has led to earlier detection of PCa and significantly reduced PCa-associated fatalities. PSA screening is currently the single best test for prostate cancer and is widely used in the diagnosis of prostate cancer, but it does not help in determining whether the detected cancer will cause clinically significant disease. Whereas PSA is an excellent marker for the follow-up of patients with established prostate cancer, some men with prostate cancer may have normal PSA levels. A moderate elevation of the PSA level (4-10 ng/mL) has a low specificity for prostate cancer, and an elevated PSA level is not specific for prostate cancer. Elevated serum PSA levels may also be associated with prostatitis, prostate infarction, PIN, prostate biopsy, transurethral resection of the prostate, and urethral catheterization.

Because of the limitations of PSA screening, there have been efforts to improve its diagnostic specificity through the use of derivative indices such as PSA density, age-related PSA levels, TZ-PSA density, PSA velocity, free PSA levels, complexed PSA (cPSA) measurements, and free-to-total PSA ratios. The free-to-total PSA ratio measures both bound and free PSA as a percentage of total PSA and is a useful additional discriminator between cancer and benign pathology, especially in patients with moderately elevated serum PSA levels (4-10 ng/mL). This ratio is also useful in determining whether a repeat biopsy is appropriate in a patient with a moderately elevated PSA level whose initial systematic biopsy results are negative. The lower the percentage of free PSA, the higher the likelihood of cancer.

Thus, development of additional serum and tissue biomarkers to supplement PSA screening is needed.

Lapointe et al. (PNAS vol. 101, no. 3, 2004, pages 811-816) looked at 5153 variably expressed genes in normal and prostate tumor tissue to classify prostate samples, and identified MUC1 and AZP1 in multivariate analysis as being strong predictors of tumor recurrence independent of tumor grade.

Fradet et al (EUROPEAN UROLOGY SUPPLEMENTS vol. 5, no. 2, 1 April 2006) assessed sensitivity, specificity and specimen informative rate of a prototype APTIMA® PCA3 assay, examining PCA3 and PSA mRNA in urine of a mixed population of prostate cancer positive and prostate cancer negative patients.

Singh et al (PROCEEDINGS OF THE ANNUAL MEETING OF THE AMERICAN ASSOCIATION FOR CANCER RESEARCH, AMERICAN ASSOCIATION FOR CANCER RESEARCH, US, vol. 47, 1 April 2006) investigated the biological significance of a reported increase in SPINK in prostate cancer, using RNA interference mediated silencing of SPINK in transfected human prostate cancer cells.

In a separate publication (CANCER LETTERS vol. 237, 2006, pages 298 - 304) Singh et al. identified 76 potential prostate cancer markers and examined their androgen dependence by determining mRNA levels of their murine orthologs in response to androgen treatment in congenital androgen-deficient mice.

Bjartell et al. studied whether tumor-associated trypsin inhibitor (TATI, PSTI, SPINK1) expression in prostate cancer is related to tumor grade (EUROPEAN UROLOGY SUPPLEMENTS, vol. 5, no. 14, 1 September 2006).

Bharathi et al (NEOPLASIA, vol. 8, no. 10, 1 October 2006) described detection of TMPRSS2:ERG fusion transcripts in urine of men with prostate cancer.

### SUMMARY OF THE INVENTION

A method, as defined in the claims, is provided for identifying prostate cancer in a patient. The method may comprise: Detecting in a sample containing prostate cells overexpression of SPINK1 compared to normal expression of SPINK1; and, detecting in the sample containing prostate cells normal expression of ERG and/or ETV1, wherein detecting in the sample containing prostate cells overexpression of SPINK1 and normal expression of ERG and/or ETV1 identifies prostate cancer in the patient. In some embodiments, the detecting step comprises detecting overexpression of SPINK1 RNA. In other embodiments, the detecting step comprises detecting overexpression of SPINK1 protein. In some embodiments, the sample containing prostate cells is prostate tissue, blood, urine, semen, prostatic secretions or isolated prostate cells. In some embodiments, detecting in the sample containing prostate cells overexpression of SPINK1 identifies invasive prostate cancer in the patient. In some embodiments, the sample containing prostate cells is from a patient following radical prostatectomy and overexpression of SPINK1 identifies recurrence of prostate cancer in the patient following radical prostatectomy.

A kit is additionally provided comprising at least one of the following: (a) a first labelled oligonucleotide probe comprising a sequence that hybridizes specifically to SPINK1 RNA or cDNA, a second labelled oligonucleotide probe comprising a sequence that hybridizes specifically to ERG RNA or cDNA, and a third labelled oligonucleotide probe comprising a sequence that hybridizes specifically to ETV1 RNA or cDNA; (b) a first pair of amplification oligonucleotides wherein each amplification oligonucleotide in the first pair comprises a sequence that hybridizes specifically to SPINK1 RNA or cDNA, a second pair of amplification oligonucleotides wherein each amplification oligonucleotide in the second pair comprises a sequence that hybridizes specifically to ERG RNA or cDNA, and a third pair of amplification oligonucleotides wherein each amplification oligonucleotide comprises a sequence that hybridizes specifically to ETV1 RNA or cDNA; or (c) a first antibody that binds specifically to SPINK1 protein, a second antibody that binds specifically to ERG protein, and a third antibody that binds specifically to ETV1 protein.

Additional embodiments, as defined in the claims, are described herein.

### DESCRIPTION OF THE FIGURES

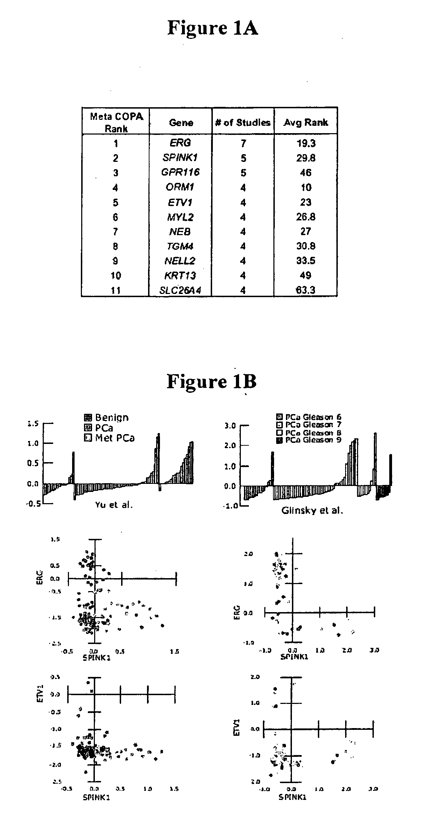

Figure 1 shows that meta COPA identified SPINK1 as a mutually exclusive outlier with ERG and ETV1 in prostate cancer. a. Genes were ranked by the number of studies in which they scored in the top 100 outliers (ranked by COPA) at any of the three pre-defined percentile cutoffs (75th, 90th, 95th). Genes were further ranked by their average COPA rank (Avg. Rank) in studies where they ranked in the top 100. b. The expression of SPINK1 and scatter plots of ERG vs. SPINK1 and ETV1 vs. SPINK1 expression are shown from two studies where SPINK1 ranked as a top 100 COPA outlier. Scatter plots are shown for ERG vs. SPINK1 (middle panels) and ETV1 vs. SPINK1 (lower panels) for all samples in both studies.

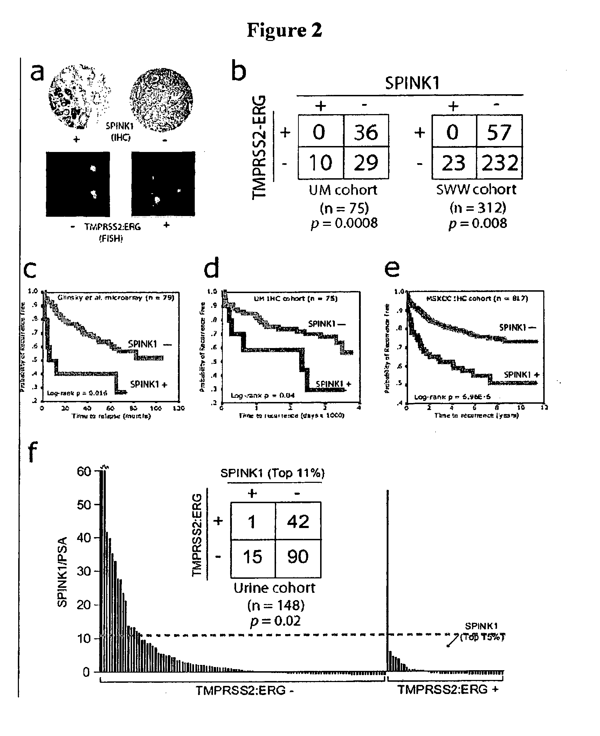

Figure 2 shows that SPINK1 over-expression identifies an aggressive subset of ETS negative prostate cancers and can be detected non-invasively. a-b. SPINK1 expression was evaluated in two cohorts (University of Michigan (UM) and Swedish Watchful Waiting (SWW)) using immunohistochemistry (IHC) on tissue microarrays that have previously been evaluated for TMRPSS2:ERG status by fluorescence *in situ* hybridization (FISH). a. Representative SPINK1 positive and negative cores are shown, along with cells from the same cores negative and positive for TMRPSS2:ERG rearrangement by FISH. c. Contingency tables for SPINK1 expression and TMRPSS2:ERG status and p-values for Fisher's exact tests for both cohorts are indicated. c-e. Relationship between SPINK1 outlier expression and biochemical recurrence after surgical resection. Kaplan-Meier analyses of outlier SPINK1 expression from the (c) Glinsky et al. DNA microarray dataset and SPINK1 IHC from the (d) UM and (e) Memorial Sloan Kettering Cancer Center (MSKCC) cohorts and biochemical recurrence after surgical resection are shown. f. Non-invasive detection of SPINK1 outlier-expression in men with TMRPSS2:ERG negative prostate cancer. Contingency table for SPINK1 outlier expression and TMPRSS2:ERG status and the Fisher's exact test p-value is shown.

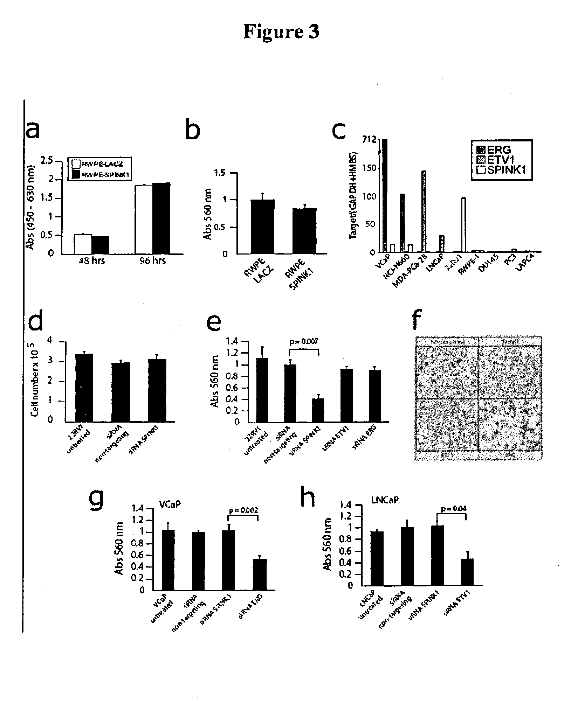

Figure 3 shows that knockdown of SPINK1 in 22RV1 prostate cancer cells attenuates invasiveness. a-b. The benign immortalized prostate cell line RWPE was infected with SPINK1 or LACZ adenovirus as indicated and assayed for (a) proliferation or (b) invasion through a modified basement membrane. c. qPCR for SPINK1, ERG and ETV1 outlier expression. d-f. SPINK1 mediates invasiveness in 22RV1 cells. Cells were assayed for (d) proliferation and (e) invasion. Photomicrographs of invaded cells treated with the indicated siRNA are shown in f. g. VCaP (TMPRSS2:ERG positive) and g) LNCaP (ETV1 rearrangement positive) prostate cancer cell lines were treated with transfection reagent alone (untreated), or transfected with non-targeting nucleic acid or siRNA against SPINK1, ETV1 or ERG as indicated and assayed for invasion.

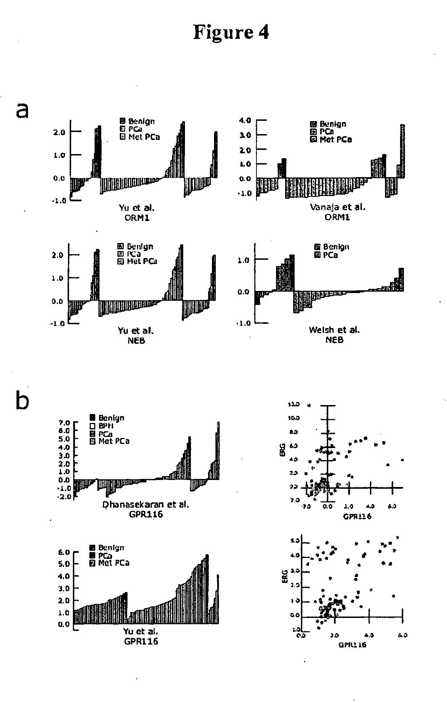

Figure 4 shows meta-outlier genes showing over-expression in benign prostate tissue and ETS positive prostate cancers. a. The expression of meta-outlier genes ORM (ranked 4th) and NEB (ranked 7th) in normalized expression units are shown from the indicated studies, according to the sample classes described in Figure 1, revealing outlier expression in multiple benign samples. b. The expression of the 3rd ranked meta-outlier gene GPR116 (left panels) and scatter plots of GPR116 vs. ERG (right panels) for all profiled samples in two studies shows co-outlier expression of GPR116 and ERG in
   multiple samples.

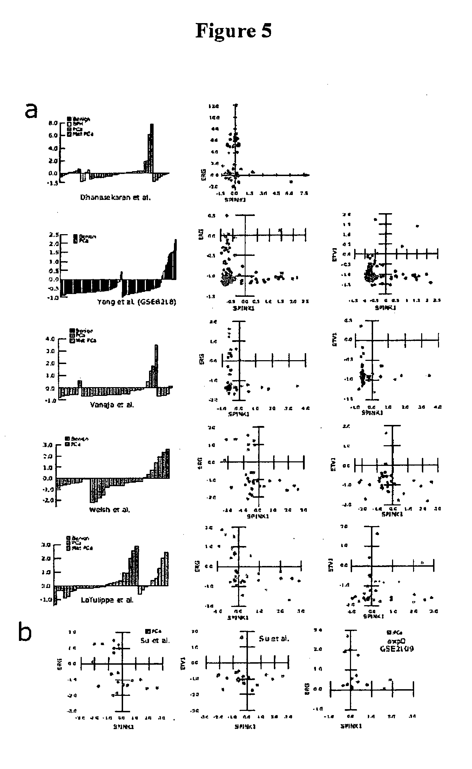

Figure 5 shows over-expression of SPINK1 in prostate cancer compared to benign prostate tissue and mutually exclusive over-expression with ERG and ETV1 in DNA microarray studies. The expression of SPINK1 and scatter plots of SPINK1 vs. ERG and SPINK1 vs. ETV1 (if measured) for five studies profiling distinct classes of prostate tissue (a) and two studies profiling prostate cancers as part of multi-cancer studies (b) are shown as in Figure 1.

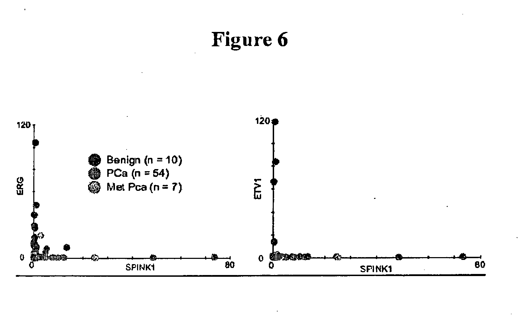

Figure 6 shows over-expression of SPINK1 in prostate cancer compared to benign prostate tissue and mutually exclusive over-expression with ERG and ETV1. a. Scatter plots of ERG vs. SPINK1 (left panel) and ETV1 vs. SPINK1 (right panel) by qPCR in 10 benign prostate samples, 54 localized prostate cancers (PCa) and 7 metastatic (Met) PCa samples.

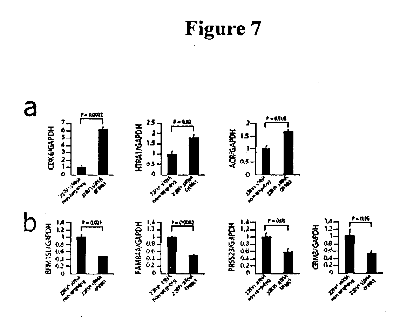

Figure 7 shows qPCR confirmation of genes differentially expressed upon SPINK1 knockdown in 22RV1 cells. Selected a) over- and b) under-expressed genes in 22RV1 siSPINK1 cells were assessed by quantitative PCR as shown.

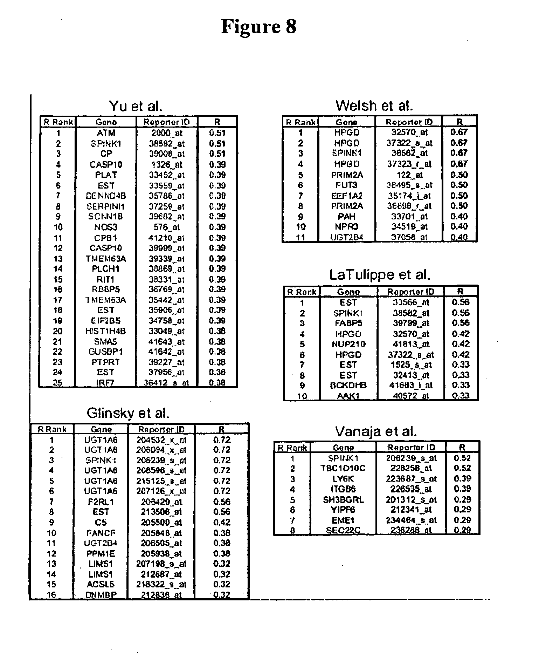

Figure 8 shows identification of genes showing co-expression with SPINK1 across multiple prostate cancer profiling studies.

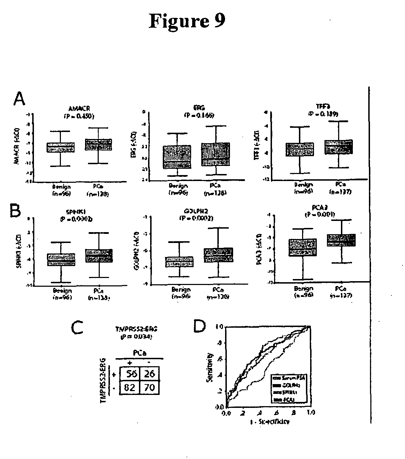

Figure 9 shows characterization of candidate urine-based biomarkers of prostate cancer. A-C. Quantitative PCR (qPCR) of whole transcriptome amplified (WTA) cDNA from urine obtained from patients presenting for needle biopsy or prostatectomy. Biomarker expression in patients with negative needle biopsies or patients with prostate cancer are shown. The -ΔCt values of genes that were not significant predictors of prostate cancer by univariate analysis (see Table 5) are shown in A, and the expression of those that were significant predictors are shown in B and C. D. Receiver operator characteristic (ROC) curves for individual variables for the diagnosis of prostate cancer.

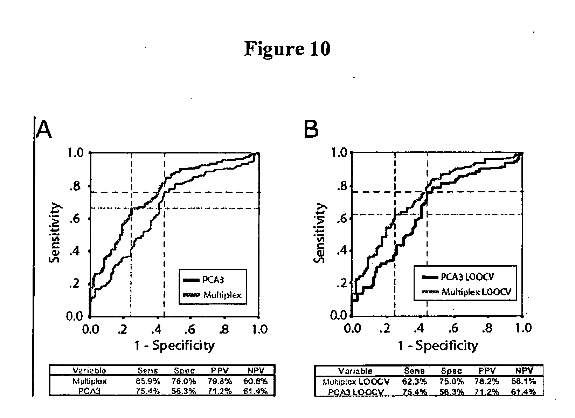

Figure 10 shows a multiplexed set of urine biomarkers out performs PCA3 alone in the detection of prostate cancer. A. Multivariate regression analysis resulted in a multiplexed model including SPINK1, PCA3, GOLPH2 and TMPRSS2:ERG as significant predictors of prostate cancer (see Table 5). The point on the ROC curve with the maximum sum of sensitivity (Sens) and specificity (Spec) is indicated by the dashed line, and the positive and negative predictive values (PPV and NPV, respectively) are given. B. As in A, except a leave-one-out cross validation (LOOCV) strategy was used to generate unbiased area under curves.

### GENERAL DESCRIPTION

When applied to the Oncomine database (Rhodes et al., Proc Natl Acad Sci USA 101, 9309 [2004]; Rhodes et al., Neoplasia 6, 1 [2004]), the methodology termed Cancer Outlier Profile Analysis (COPA) correctly identified several known oncogenes, including PBX1 in leukemia and CCND1 in multiple myeloma (Tomlins et al., Science 310, 644 [2005]). In addition, COPA nominated ETS family genes as candidate oncogenes in prostate cancer prompting the discovery of recurrent chromosomal rearrangements involving ERG or ETV1 and the androgen-regulated gene TMPRSS2 (Tomlins et al., [2005], supra).

As 50-70% of prostate cancers harbor TMPRSS2:ETS gene fusions, experiments were conducted to identify additional candidate oncogenes in prostate cancers. Experiments conducted used a meta-analysis of COPA applied to 7 prostate cancer profiling studies and analyzed candidates for outlier expression in prostate cancer and mutually exclusive over-expression with ERG and ETV1. SPINK1, which was identified as the 2nd ranked meta-outlier, met both criteria across 8 data sets. SPINK1 showed marked overexpression in 50 of 325 (15.4%) profiled prostate cancers, but only 1 of 56 (1.8%) benign prostate tissue samples. In all 325 profiled prostate cancer samples, SPINK1, ERG and ETV1 showed mutually exclusive outlier expression. The over-expression of SPINK1 in a fraction of cancer samples compared to benign prostate tissue and the mutually exclusive over-expression of SPINK1, ERG and ETV1 was confirmed by quantitative PCR. Fluorescence *in situ* hybridization from tissue from one of the localized prostate cancers over-expressing SPINK1 did not reveal gene rearrangements or amplification, indicating that SPINK1 is up-regulated through increased transcription. Together these results, consistent across different assays, microarray platforms, laboratories and sample cohorts, demonstrate that SPINK1 is exclusively over-expressed in prostate cancers without TMPRSS2:ETS gene fusions (as indicated by ERG or ETV1 over-expression).

Further experiments demonstrated that SPINK1 outlier expression was correlated with an increase in prostate cancer recurrence (e.g., following surgery). Accordingly, in some embodiments, the methods of screening samples to determine likelihood of prostate cancer recurrence are provided. Subjects at increased risk of recurrence can then be offered more aggressive treatment or additional therapies. Conversely, subjects found to be at decreased risk of recurrence or lacking increased risk of recurrence can be spared the side effects of unnecessary treatments.

### DEFINITIONS

To facilitate an understanding of the disclosure and claims described herein, a number of terms and phrases are defined below:
As used herein, the term "outlier expression of SPINK1" refers to an altered level of expression of SPINK1 nucleic acid (e.g., mRNA) or protein relative to the level normally found (e.g., the level in a subject not diagnosed with cancer). In some embodiments, normal levels are the average level in a population of one or more individuals not diagnosed with cancer. In other embodiments, normal levels are determined within other tissues of the individual to be diagnosed. In some embodiments, expression is altered by at least 10%, preferably at least 20%, even more preferably at least 50%, yet more preferably at least 75%, still more preferably at least 90%, and most preferably at least 100% relative to the level of expression normally found (e.g., in non-cancerous tissue). Expression levels may be determined using any suitable method, including, but not limited to, those disclosed herein (e.g., Example 1 below). In some embodiments, samples positive for outlier expression of SPINK1 are those whose expression differs by greater than about 0.1, preferably greater than 0.2, and even more preferably greater than 0.5 normalized expression units. Normalized expression units may be calculated using any suitable method, including, but not limited to, those described the experimental section below.

As used herein, the term "overexpression of SPINK1" refers to a higher level of expression of SPINK1 nucleic acid (e.g., mRNA) or protein relative to the level normally found. In some embodiments, expression is increased at least 10%, preferably at least 20%, even more preferably at least 50%, yet more preferably at least 75%, still more preferably at least 90%, and most preferably at least 100% relative to the level of expression normally found. The level of expression normally found may be determined using any number of suitable parameters. Examples include, but are not limited to, the level in non-cancerous prostate (e.g., an average of the level of SPINK1 expression in prostate tissues from multiple subjects not diagnosed with prostate cancer), the level in non-cancerous tissues (e.g., an average of the level of SPINK1 expression in non-prostate tissues from multiple subjects not diagnosed with cancer), the level in non-cancerous prostate cell lines, or a relative level of expression (e.g., the level over time in the same individual). Expression levels may be determined using any suitable method, including, but not limited to, those disclosed herein. In some embodiments, expression levels are compared to the level of expression of a known gene (e.g., the level of expression or the relative expression). In some embodiments, the known gene is PSA.

As used herein, the term "gene expression associated with prostate cancer recurrence" refers to a gene expression profile (e.g., outlier expression of SPINK1) associated with prostate cancer recurrence (e.g., in the prostate or metastatic) following treatment (e.g., surgery) for a primary tumor. In some embodiments, prostate cancer recurrence is increased at least 10%, preferably at least 20%, even more preferably at least 50%, yet more preferably at least 75%, still more preferably at least 90%, and most preferably at least 100% relative to the level of recurrence in representative subject population (e.g., average of a large population (e.g., one or more, preferably 100 or more, even more preferably 1000 or more and still more preferably 10,000 or more subjects) of subjects lacking "outlier expression of SPINK1").

The term "epitope" as used herein refers to that portion of an antigen that makes contact with a particular antibody.

When a protein or fragment of a protein is used to immunize a host animal, numerous regions of the protein may induce the production of antibodies which bind specifically to a given region or three-dimensional structure on the protein; these regions or structures are referred to as "antigenic determinants". An antigenic determinant may compete with the intact antigen *(i.e.,* the "immunogen" used to elicit the immune response) for binding to an antibody.

The terms "specific binding" or "specifically binding" when used in reference to the interaction of an antibody and a protein or peptide means that the interaction is dependent upon the presence of a particular structure *(i.e.,* the antigenic determinant or epitope) on the protein; in other words the antibody is recognizing and binding to a specific protein structure rather than to proteins in general. For example, if an antibody is specific for epitope "A," the presence of a protein containing epitope A (or free, unlabelled A) in a reaction containing labeled "A" and the antibody will reduce the amount of labeled A bound to the antibody.

As used herein, the terms "non-specific binding" and "background binding" when used in reference to the interaction of an antibody and a protein or peptide refer to an interaction that is not dependent on the presence of a particular structure (*i.e*., the antibody is binding to proteins in general rather that a particular structure such as an epitope).

As used herein, the term "subject" refers to any animal (*e.g*., a mammal), including, but not limited to, humans, non-human primates, rodents, and the like, which is to be the recipient of a particular treatment. Typically, the terms "subject" and "patient" are used interchangeably herein in reference to a human subject.

As used herein, the term "subject suspected of having cancer" refers to a subject that presents one or more symptoms indicative of a cancer (*e.g*., a noticeable lump or mass) or is being screened for a cancer (*e.g*., during a routine physical). A subject suspected of having cancer may also have one or more risk factors. A subject suspected of having cancer has generally not been tested for cancer. However, a "subject suspected of having cancer" encompasses an individual who has received an initial diagnosis (*e.g*., a CT scan showing a mass or increased PSA level) but for whom the stage of cancer is not known. The term further includes people who once had cancer (*e.g*., an individual in remission).

As used herein, the term "characterizing cancer in subject" refers to the identification of one or more properties of a cancer sample in a subject, including but not limited to, the presence of benign, pre-cancerous or cancerous tissue, the stage of the cancer, and the subject's prognosis. Cancers may be characterized by the identification of the expression of one or more cancer marker genes, including but not limited to, the cancer markers disclosed herein.

As used herein, the term "characterizing prostate tissue in a subject" refers to the identification of one or more properties of a prostate tissue sample (*e.g*., including but not limited to, the presence of cancerous tissue, the presence of pre-cancerous tissue that is likely to become cancerous, and the presence of cancerous tissue that is likely to metastasize). Tissues may be characterized by the identification of the expression of one or more cancer marker genes, including but not limited to, the cancer markers disclosed herein.

As used herein, the term "cancer marker genes" refers to a gene whose expression level, alone or in combination with other genes, is correlated with cancer or prognosis of cancer. The correlation may relate to either an increased or decreased expression of the gene. For example, the expression of the gene may be indicative of cancer, or lack of expression of the gene may be correlated with poor prognosis in a cancer patient.

As used herein, the term "a reagent that specifically detects the presence or absence of a cancer marker" refers to reagents used to detect the expression of one or more cancer markers (*e.g.*, including but not limited to, the cancer markers described herein). Examples of suitable reagents include but are not limited to, nucleic acid probes capable of specifically hybridizing to the gene of interest, PCR primers capable of specifically amplifying the gene of interest, and antibodies capable of specifically binding to proteins expressed by the gene of interest. Other non-limiting examples can be found in the description and examples below.

As used herein, the term "instructions for using said kit for detecting cancer in said subject" includes instructions for using the reagents contained in the kit for the detection and characterization of cancer in a sample from a subject. The instructions may further comprise the statement of intended use required by the U.S. Food and Drug Administration (FDA) in labeling *in vitro* diagnostic products.

As used herein, the term "stage of cancer" refers to a qualitative or quantitative assessment of the level of advancement of a cancer. Criteria used to determine the stage of a cancer include, but are not limited to, the size of the tumor, whether the tumor has spread to other parts of the body and where the cancer has spread *(e.g.,* within the same organ or region of the body or to another organ).

As used herein, the term "providing a prognosis" refers to providing information regarding the impact of the presence of cancer (*e.g*., as determined by the diagnostic methods described herein) on a subject's future health (*e.g*., expected morbidity or mortality, the likelihood of getting cancer, and the risk of metastasis).

As used herein, the term "initial diagnosis" refers to results of initial cancer diagnosis (*e.g*. the presence or absence of cancerous cells). An initial diagnosis does not include information about the stage of the cancer of the risk of prostate specific antigen failure.

As used herein, the term "biopsy tissue" refers to a sample of tissue (*e.g.*, prostate tissue) that is removed from a subject for the purpose of determining if the sample contains cancerous tissue. Biopsy tissue may be obtained because a subject is suspected of having cancer. The biopsy tissue is then examined (*e.g.,* by microscopy) for the presence or absence of cancer.

As used herein, the term "non-human animals" refers to all non-human animals including, but are not limited to, vertebrates such as rodents, non-human primates, ovines, bovines, ruminants, lagomorphs, porcines, caprines, equines, canines, felines, aves, etc.

The term "gene" refers to a nucleic acid (*e.g.*, DNA) sequence that comprises coding sequences necessary for the production of a polypeptide, precursor, or RNA (*e.g*., rRNA, tRNA). The polypeptide can be encoded by a full length coding sequence or by any portion of the coding sequence so long as the desired activity or functional properties (*e.g*., enzymatic activity, ligand binding, signal transduction, immunogenicity, etc.) of the full-length or fragment are retained. The term also encompasses the coding region of a structural gene and the sequences located adjacent to the coding region on both the 5' and 3' ends for a distance of about 1 kb or more on either end such that the gene corresponds to the length of the full-length mRNA. Sequences located 5' of the coding region and present on the mRNA are referred to as 5' non-translated sequences. Sequences located 3' or downstream of the coding region and present on the mRNA are referred to as 3' non-translated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

As used herein, the term "heterologous gene" refers to a gene that is not in its natural environment. For example, a heterologous gene includes a gene from one species introduced into another species. A heterologous gene also includes a gene native to an organism that has been altered in some way (*e.g*., mutated, added in multiple copies, linked to non-native regulatory sequences, etc). Heterologous genes are distinguished from endogenous genes in that the heterologous gene sequences are typically joined to DNA sequences that are not found naturally associated with the gene sequences in the chromosome or are associated with portions of the chromosome not found in nature (*e.g*., genes expressed in loci where the gene is not normally expressed).

As used herein, the term "gene expression" refers to the process of converting genetic information encoded in a gene into RNA (*e.g*., mRNA, rRNA, tRNA, or snRNA) through "transcription" of the gene (*i.e.,* via the enzymatic action of an RNA polymerase), and for protein encoding genes, into protein through "translation" of mRNA. Gene expression can be regulated at many stages in the process. "Up-regulation" or "activation" refers to regulation that increases the production of gene expression products (*i.e.*, RNA or protein), while "down-regulation" or "repression" refers to regulation that decrease production. Molecules (*e.g*., transcription factors) that are involved in up-regulation or down-regulation are often called "activators" and "repressors," respectively.

In addition to containing introns, genomic forms of a gene may also include sequences located on both the 5' and 3' end of the sequences that are present on the RNA transcript. These sequences are referred to as "flanking" sequences or regions (these flanking sequences are located 5' or 3' to the non-translated sequences present on the mRNA transcript). The 5' flanking region may contain regulatory sequences such as promoters and enhancers that control or influence the transcription of the gene. The 3' flanking region may contain sequences that direct the termination of transcription, post-transcriptional cleavage and polyadenylation.

As used herein, the term "oligonucleotide," refers to a short length of single-stranded polynucleotide chain. Oligonucleotides are typically less than 200 residues long (*e.g*., between 15 and 100), however, as used herein, the term is also intended to encompass longer polynucleotide chains. Oligonucleotides are often referred to by their length. For example a 24 residue oligonucleotide is referred to as a "24-mer". Oligonucleotides can form secondary and tertiary structures by self-hybridizing or by hybridizing to other polynucleotides. Such structures can include, but are not limited to, duplexes, hairpins, cruciforms, bends, and triplexes.

As used herein, the terms "complementary" or "complementarity" are used in reference to polynucleotides (*i.e.,* a sequence of nucleotides) related by the base-pairing rules. For example, the sequence "5'-A-G-T-3'," is complementary to the sequence "3'-T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids.

The term "homology" refers to a degree of complementarity. There may be partial homology or complete homology (*i.e.,* identity). A partially complementary sequence is a nucleic acid molecule that at least partially inhibits a completely complementary nucleic acid molecule from hybridizing to a target nucleic acid is "substantially homologous." The inhibition of hybridization of the completely complementary sequence to the target sequence may be examined using a hybridization assay (Southern or Northern blot, solution hybridization and the like) under conditions of low stringency. A substantially homologous sequence or probe will compete for and inhibit the binding (*i.e.*, the hybridization) of a completely homologous nucleic acid molecule to a target under conditions of low stringency. This is not to say that conditions of low stringency are such that non-specific binding is permitted; low stringency conditions require that the binding of two sequences to one another be a specific (*i.e.*, selective) interaction. The absence of non-specific binding may be tested by the use of a second target that is substantially non-complementary (*e.g*., less than about 30% identity); in the absence of non-specific binding the probe will not hybridize to the second non-complementary target.

When used in reference to a double-stranded nucleic acid sequence such as a cDNA or genomic clone, the term "substantially homologous" refers to any probe that can hybridize to either or both strands of the double-stranded nucleic acid sequence under conditions of low stringency as described above.

When used in reference to a single-stranded nucleic acid sequence, the term "substantially homologous" refers to any probe that can hybridize (*i.e.,* it is the complement of) the single-stranded nucleic acid sequence under conditions of low stringency as described above.

As used herein, the term "hybridization" is used in reference to the pairing of complementary nucleic acids. Hybridization and the strength of hybridization (*i.e.*, the strength of the association between the nucleic acids) is impacted by such factors as the degree of complementary between the nucleic acids, stringency of the conditions involved, the Tₘ of the formed hybrid, and the G:C ratio within the nucleic acids. A single molecule that contains pairing of complementary nucleic acids within its structure is said to be "self-hybridized."

As used herein, the term "Tₘ" is used in reference to the "melting temperature." The melting temperature is the temperature at which a population of double-stranded nucleic acid molecules becomes half dissociated into single strands. The equation for calculating the Tₘ of nucleic acids is well known in the art. As indicated by standard references, a simple estimate of the Tₘ value may be calculated by the equation: Tₘ = 81.5 + 0.41(% G + C), when a nucleic acid is in aqueous solution at 1 M NaCl (See *e.g*., Anderson and Young, Quantitative Filter Hybridization, in Nucleic Acid Hybridization [1985]). Other references include more sophisticated computations that take structural as well as sequence characteristics into account for the calculation of Tₘ.

As used herein the term "stringency" is used in reference to the conditions of temperature, ionic strength, and the presence of other compounds such as organic solvents, under which nucleic acid hybridizations are conducted. Under "low stringency conditions" a nucleic acid sequence of interest will hybridize to its exact complement, sequences with single base mismatches, closely related sequences (*e.g*., sequences with 90% or greater homology), and sequences having only partial homology (*e.g*., sequences with 50-90% homology). Under 'medium stringency conditions," a nucleic acid sequence of interest will hybridize only to its exact complement, sequences with single base mismatches, and closely relation sequences (*e.g.,* 90% or greater homology). Under "high stringency conditions," a nucleic acid sequence of interest will hybridize only to its exact complement, and (depending on conditions such a temperature) sequences with single base mismatches. In other words, under conditions of high stringency the temperature can be raised so as to exclude hybridization to sequences with single base mismatches.

"High stringency conditions" when used in reference to nucleic acid hybridization comprise conditions equivalent to binding or hybridization at 42°C in a solution consisting of 5X SSPE (43.8 g/l NaCl, 6.9 g/l NaH₂PO₄ H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 0.1X SSPE, 1.0% SDS at 42°C when a probe of about 500 nucleotides in length is employed.

"Medium stringency conditions" when used in reference to nucleic acid hybridization comprise conditions equivalent to binding or hybridization at 42°C in a solution consisting of 5X SSPE (43.8 g/l NaCl, 6.9 g/l NaH₂PO₄ H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 1.0X SSPE, 1.0% SDS at 42°C when a probe of about 500 nucleotides in length is employed.

"Low stringency conditions" comprise conditions equivalent to binding or hybridization at 42°C in a solution consisting of 5X SSPE (43.8 g/l NaCl, 6.9 g/l NaH₂PO₄ H₂O and 1.85 g/l EDTA, pH adjusted to 7.4 with NaOH), 0.1% SDS, 5X Denhardt's reagent [50X Denhardt's contains per 500 ml: 5 g Ficoll (Type 400, Pharamcia), 5 g BSA (Fraction V; Sigma)] and 100 µg/ml denatured salmon sperm DNA followed by washing in a solution comprising 5X SSPE, 0.1% SDS at 42°C when a probe of about 500 nucleotides in length is employed.

The art knows well that numerous equivalent conditions may be employed to comprise low stringency conditions; factors such as the length and nature (DNA, RNA, base composition) of the probe and nature of the target (DNA, RNA, base composition, present in solution or immobilized, etc.) and the concentration of the salts and other components (*e.g*., the presence or absence of formamide, dextran sulfate, polyethylene glycol) are considered and the hybridization solution may be varied to generate conditions of low stringency hybridization different from, but equivalent to, the above listed conditions. In addition, the art knows conditions that promote hybridization under conditions of high stringency (*e.g*., increasing the temperature of the hybridization and/or wash steps, the use of formamide in the hybridization solution, etc.) (see definition above for "stringency").

As used herein, the term "probe" refers to an oligonucleotide (*i.e.,* a sequence of nucleotides), whether occurring naturally as in a purified restriction digest or produced synthetically, recombinantly or by PCR amplification, that is capable of hybridizing to at least a portion of another oligonucleotide of interest. A probe may be single-stranded or double-stranded. Probes are useful in the detection, identification and isolation of particular gene sequences. It is contemplated that any probe used herein will be labeled with any "reporter molecule," so that is detectable in any detection system, including, but not limited to enzyme (*e.g*., ELISA, as well as enzyme-based histochemical assays), fluorescent, radioactive, and luminescent systems. It is not intended that the described methods be limited to any particular detection system or label.

As used herein the term "portion" when in reference to a nucleotide sequence (as in "a portion of a given nucleotide sequence") refers to fragments of that sequence. The fragments may range in size from four nucleotides to the entire nucleotide sequence minus one nucleotide (10 nucleotides, 20, 30, 40, 50, 100, 200, etc.).

"Amino acid sequence" and terms such as "polypeptide" or "protein" are not meant to limit the amino acid sequence to the complete, native amino acid sequence associated with the recited protein molecule.

The term "native protein" as used herein to indicate that a protein does not contain amino acid residues encoded by vector sequences; that is, the native protein contains only those amino acids found in the protein as it occurs in nature. A native protein may be produced by recombinant means or may be isolated from a naturally occurring source.

As used herein the term "portion" when in reference to a protein (as in "a portion of a given protein") refers to fragments of that protein. The fragments may range in size from four amino acid residues to the entire amino acid sequence minus one amino acid.

The terms "overexpression" and "overexpressing" and grammatical equivalents, are used in reference to levels of mRNA to indicate a level of expression approximately 3-fold higher (or greater) than that observed in a given tissue in a control or non-transgenic animal. Levels of mRNA are measured using any of a number of techniques known to those skilled in the art including, but not limited to Northern blot analysis. Appropriate controls are included on the Northern blot to control for differences in the amount of RNA loaded from each tissue analyzed (*e.g*., the amount of 28S rRNA, an abundant RNA transcript present at essentially the same amount in all tissues, present in each sample can be used as a means of normalizing or standardizing the mRNA-specific signal observed on Northern blots). The amount of mRNA present in the band corresponding in size to the correctly spliced transgene RNA is quantified; other minor species of RNA which hybridize to the transgene probe are not considered in the quantification of the expression of the transgenic mRNA.

As used herein, the term *"in vitro"* refers to an artificial environment and to processes or reactions that occur within an artificial environment. In vitro environments can consist of, but are not limited to, test tubes and cell culture. The term "*in vivo"* refers to the natural environment *(e.g.,* an animal or a cell) and to processes or reaction that occur within a natural environment.

The terms "test compound" and "candidate compound" refer to any chemical entity, pharmaceutical, drug, and the like that is a candidate for use to treat or prevent a disease, illness, sickness, or disorder of bodily function (*e.g*., cancer). Test compounds comprise both known and potential therapeutic compounds. A test compound can be determined to be therapeutic by screening using the screening methods described herein. Test compounds may include antisense compounds.

As used herein, the term "sample" is used in its broadest sense. In one sense, it is meant to include a specimen or culture obtained from any source, as well as biological and environmental samples. Biological samples may be obtained from animals (including humans) and encompass fluids, solids, tissues, and gases. Biological samples include blood products, such as plasma, serum and the like. Environmental samples include environmental material such as surface matter, soil, water, crystals and industrial samples. Such examples are not however to be construed as limiting the sample types applicable to the described compositions and methods.

As used herein, the term "prostate sample" refers to any sample containing prostate cells or secretions. Example of prostate samples include, but are not limited to, a prostate tissue sample (e.g., a biopsy sample) or a urine sample.

As used herein, the terms "detect", "detecting", or "detection" may describe either the general act of discovering or discerning or the specific observation of a detectably labeled composition.

As used herein, the term "siRNAs" refers to small interfering RNAs. siRNAs may comprise a duplex, or double-stranded region, of about 18-25 nucleotides long; often siRNAs contain from about two to four unpaired nucleotides at the 3' end of each strand. At least one strand of the duplex or double-stranded region of a siRNA is substantially homologous to, or substantially complementary to, a target RNA molecule. The strand complementary to a target RNA molecule is the "antisense strand;" the strand homologous to the target RNA molecule is the "sense strand," and is also complementary to the siRNA antisense strand. siRNAs may also contain additional sequences; non-limiting examples of such sequences include linking sequences, or loops, as well as stem and other folded structures. siRNAs appear to function as key intermediaries in triggering RNA interference in invertebrates and in vertebrates, and in triggering sequence-specific RNA degradation during posttranscriptional gene silencing in plants.

The term "RNA interference" or "RNAi" refers to the silencing or decreasing of gene expression by siRNAs. It is the process of sequence-specific, post-transcriptional gene silencing in animals and plants, initiated by siRNA that is homologous in its duplex region to the sequence of the silenced gene. The gene may be endogenous or exogenous to the organism, present integrated into a chromosome or present in a transfection vector that is not integrated into the genome. The expression of the gene is either completely or partially inhibited. RNAi may also be considered to inhibit the function of a target RNA; the function of the target RNA may be complete or partial.

### DETAILED DESCRIPTION OF THE INVENTION

Compositions and methods for cancer research, diagnosis, and treatment, including but not limited to, cancer markers are described. In particular, SPINK1 and other markers for prostate cancer are disclosed. Accordingly, methods and kits for the detection of markers, as well as drug screening and therapeutic applications are disclosed.

### I. Prostate Cancer Markers

Markers whose expression is specifically altered in cancerous prostate tissues are provided. Such markers find use in the diagnosis and characterization of prostate cancer. For example, experiments described herein identified SPINK1 as being overexpressed in prostate cancer. In addition, SPINK1, ERG and ETV1 showed mutually exclusive outlier expression. Further experiments identified a multiplex panel assay for detection of gene expression related to prostate cancer including SPINK1, PCA3, GOLPH2 and TMPRSS2:ERG fusions.

### A. SPINK1

The cDNA sequence of SPINK1 (serine peptidase inhibitor, Kazal type 1) is provided in Genbank accession number NM_003122.2. The peptide encoded by SPINK1, also known as PSTI or TATI, was originally isolated from bovine pancreas and human pancreatic juice; its normal function is thought to be the inhibition of trypsin in the pancreas (Haverback et al., Am J Med 29,421-33 (1960); Kazal et al., Journal of the American Chemical Society 70, 3034-3040 (1948); Paju et al., Crit Rev Clin Lab Sci 43, 103-42 (2006); Greene et al., Methods Enzymol 45, 813-25 (1976)). SPINK1 mRNA and protein have been detected in a variety of benign and cancerous tissues, however its expression in prostate has not been described (reviewed in Paju and Stenman, Crit Rev Clin Lab Sci 43, 103-42 (2006), Stenman, Clin Chem 48, 1206-9 (2002)). SPINK1 encodes a 79 amino acid peptide with a 23 amino acid signal peptide and is detectable in the urine and serum of healthy individuals (Paju and Stenman, supra). In addition to being strongly elevated during severe inflammation and pancreatitis, serum levels of SPINK1 may be dysregulated in numerous cancers, including pancreatic, gastric, liver, lung, breast, bladder, renal, head and neck, colorectal, kidney and ovarian cancer (reviewed in Paju and Stenman, supra, Stenman, supra). In conclusion, using a bioinformatics based approach, this study identified marked over-expression of SPINK1 in a subset of TMPRSS2:ETS negative prostate cancers, and confirmed these results using qPCR. Together, these results indicate that SPINK1 over-expression plays a role in prostate cancer development as well as serving as a biomarker for a molecular subtype of prostate cancer.

### B. ERG

ERG (NM_004449), has been demonstrated to be highly expressed in prostate epithelium relative to other normal human tissues. The ERG gene is located on chromosome 21. The gene is located at 38,675,671- 38,955,488 base pairs from the pter. The ERG gene is 279,817 total bp; minus strand orientation. The corresponding ERG cDNA and protein sequences are given at GenBank accesssion no. M17254 and GenBank accession no. NP04440 (Swiss Protein acc. no. P11308), respectively.

### C. ETV1

The ETV1 gene is located on chromosome 7 (GenBank accession nos. NC_000007.11; NC_086703.11; and NT_007819.15). The gene is located at 13,708330 - 13,803,555 base pairs from the pter. The ETV1 gene is 95,225 bp total, minus strand orientation. The corresponding ETV1 cDNA and protein sequences are given at GenBank accession nos. NM_004956 and NP_004947 (Swiss protein acc. no. P50549), respectively.

### D. PCA3

The gene for PCA3, also known as DD3 (Bussemakers, PCT Publication No. WO 98/45420, Schalken, Eur. Urol. 34(suppl. 3):3-6 (1998), Bussemakers et al., Cancer Res. 59:5975-5979 (1999) and Bussemakers, Eur. Urol. 35:408-412 (1999)) is located on chromosome 9 and more precisely to region 9q21-22. It consists of four exons, which give rise, by both alternative splicing and alternative poly-adenylation, to differently sized transcripts. By RT-PCR, PCA3^{dd3} expression was found to be limited to the prostate tissue and absent in all other tissues tested, including testis, seminal vesicle, ovary, placenta and bladder. In addition Northern blot analysis showed that PCA3^{dd3} is highly expressed in the vast majority of prostate cancer examined (47 out of 50) whereas no or very low expression is detected in BPH or normal prostate cells from the same patients. There is at least a 20-fold over-expression of PCA3^{dd3} in prostate carcinoma in comparison to normal or BPH tissues. PCA3^{dd3} expression seems to increase with tumor grade and is detected in metastatic lesions.

PCA3 is a gene wherein significant alternative splicing (as well as alternative poly-adenylation) occurs, as evidenced by the differently sized transcripts observed on Northern blots and the different types of clones identified. Virtually every combination of exons is possible.

From the 80 analyzed cDNA clones, at least four different transcripts were shown to be present due to alternative splicing or alternative polyadenylation (See e.g., US Patent No. 7,008,765, PCT Publication No. WO05/003387A2, US Patent No. 7,138,235 and de Kok et al., Cancer Res 2002;62:2695-8). Sequence analysis of the genomic clones as compared to the cDNA clones revealed the genomic structure of the PCA3 gene. Three introns and 4 exons are present. The first intron is approximately 20 kb in length.

The first cDNA species is found in approximately 5% of the cDNA clones and contains exons 1, 2, 3, 4a and 4b (poly-adenylation after 4b is preceeded by a real consensus poly-A-addition signal)

The second cDNA species, found in approximately 15% of the cDNA cloned, contains exons 1, 3, 4a, 4b and 4c, arises by alternative splicing of the second exon (not present in this cDNA) and terminates at a different (real consensus) poly-A-addition signal.

The third cDNA species contains exons 1, 3, 4a, and 4b and is the most common one found (approximately 65% of 80 clones) (FIG. 1). This cDNA is most likely responsible for the most prominent transcript seen by Northern blot analysis.

The fourth cDNA species detected contains exons 1, 3, and 4a representing about 15% of clones, and terminates after 4a, which is the original DD3 clone stop site. The poly-A-addition signal present here is close to the consensus sequence.

### E. GOLPH2

GOLPH2 (GP73) is a glycoprotein marker of hepatitis B associated with liver cancer (Block et al., (2005) Proc. Natl. Acad. Sci. USA, 102, 779-784). GP73 is a type II Golgi transmembrane protein that is expressed at high level in the hepatocytes of patients with viral hepatitis (Kladney, et al., 2000, Gene 249, 53-65). GP73 is constitutively expressed in biliary epithelial cells, and minimally expressed in normal hepatocytes. In contrast, livers of patients with giant-cell hepatitis display strong immunoreactivity to GP73 in multinucleated hepatocytes. GP73 mRNA and protein are expressed in highly differentiated HepG2 hepatoma cells after infection with viruses, including adenoviruses. Because GP73 is a Golgi transmembrane protein, it is not expected to exist in significant amounts in the serum, even in subjects with damaged or diseased livers.

Significant increases in whole-organ levels of GP73 have been found in liver disease due to viral causes (HBV, HCV) or nonviral causes (alcohol-induced liver disease, autoimmune hepatitis) (Kladney, et al., 2002, Hepatology 35(6):1431-40). Hepatocyte expression of GP73 is unregulated in diseased livers, regardless of etiology, whereas biliary epithelial cell expression does not change appreciably. Gp73 has been used as a marker for hepotocellular cancer (US 20050112711).

### F. TMPRSS2:ERG

TMPRSS2:ERG and other gene fusion markers of prostate cancer are described in US Publication No. US 20070212702 A1. TMPRSS2 (NM_005656), has been demonstrated to be highly expressed in prostate epithelium relative to other normal human tissues (Lin et al., Cancer Research 59: 4180 (1999)). The TMPRSS2 gene is located on chromosome 21. This gene is located at 41,750,797 - 41,801,948 bp from the pter (51,151 total bp; minus strand orientation). The human TMPRSS2 protein sequence may be found at GenBank accession no. AAC51784 (Swiss Protein accession no. 015393) and the corresponding cDNA at GenBank accession no. U75329 (see also, Paoloni-Giacobino, et al., Genomics 44: 309 (1997)).

### II. Antibodies

SPINK1 or other cancer marker polypeptides, which include fragments, derivatives and analogs thereof may be used as immunogens to produce antibodies useful for diagnostic and therapeutic applications. Such antibodies may be polyclonal or monoclonal, chimeric, humanized, single chain or Fab fragments, which may be labeled or unlabeled, all of which may be produced by using well known procedures and standard laboratory practices. *See, e.g.,* Burns, ed., Immunochemical Protocols, 3rd ed., Humana Press (2005); Harlow and Lane, Antibodies: A Laboratory Manual, Cold Spring Harbor Laboratory (1988); Kozbor et al., Immunology Today 4: 72 (1983); Köhler and Milstein, Nature 256: 495 (1975).

In some embodiments, compositions and methods utilize commercially available antibodies. Antibodies to SPINK1 are available, for example, from GeneTex, San Antonio, Tx; Novus Biologicals, Littleton, CO; Santa Cruz Biotechnology, Santa Cruz, California; and Abnova Corporation, Taipei City, Taiwan. Antibodies to SLC22A3 are available, for example, from Abnova Corporation, Taipei City, Taiwan.

### III. Diagnostic Applications

Methods for detection of expression of cancer markers (*e.g.,* SPINK1) are provided. Detection methods measure the level of cancer marker detected (e.g., in comparison to the level of the cancer marker in normal prostate tissue).

In some embodiments, expression is measured directly (*e.g*., at the RNA or protein level). In some embodiments, expression is detected in tissue samples (*e.g*., biopsy tissue). In other embodiments, expression is detected in bodily fluids (*e.g*., including but not limited to, plasma, serum, whole blood, mucus, and urine). In certain embodiments, the presence of a cancer marker is used to provide a prognosis to a subject. For example, the detection of overexpression of SPINK1 is indicative of gene expression associated with prostate cancer, outlier expression of SPINK1 is associated with prostate cancer recurrence in a subject. The information provided is also used to select a treatment course of action. For example, if a subject is found to have a marker indicative of a highly metastasizing tumor, additional therapies *(e.g.,* hormonal or radiation therapies) can be started at an earlier point when they are more likely to be effective (*e.g*., before metastasis). In addition, if a subject is found to have a tumor that is not responsive to hormonal therapy, the expense and inconvenience of such therapies can be avoided.

The cancer markers (*e.g*., SPINK1) described herein may be detected along with other markers in a multiplex or panel format. Markers are selected for their predictive value alone or in combination with SPINK1. Two or more of SPINK1, PCA3, GOLPH2 and TMPRSS2:ERG may be detected in a multiplex assay. Other exemplary prostate cancer markers include, but are not limited to: AMACR/P504S-(U.S. Pat. No: 6,262,245); PCA3 (U.S. Pat. No. 7,008,765); PCGEM1 (U.S. Pat. No. 6,828,429); prostein/P501S, P503S, P504S, P509S, P510S, prostase/P703P, P710P (U.S. Publication No. 20030185830); and, those disclosed in U.S. Pat. Nos. 5,854,206 and 6,034,218, and U.S. Publication No. 20030175736. Markers for other cancers, diseases, infections, and metabolic conditions are also contemplated for inclusion in a multiplex of panel format.

The diagnostic methods described herein may also be modified with reference to data correlating particular cancer marker(s) with the stage, aggressiveness or progression of the disease or the presence or risk of metastasis. Ultimately, the information provided by the methods described herein will assist a physician in choosing the best course of treatment for a particular patient.

### A. Sample

Any patient sample suspected of containing the cancer marker may be tested according to the methods described herein. By way of non-limiting examples, the sample may be tissue (e.g., a prostate biopsy sample or a tissue sample obtained by prostatectomy), blood, urine, semen, prostatic secretions or a fraction thereof (e.g., plasma, serum, urine supernatant, urine cell pellet or prostate cells). A urine sample is preferably collected immediately following an attentive digital rectal examination (DRE), which causes prostate cells from the prostate gland to shed into the urinary tract.

The patient sample typically requires preliminary processing designed to isolate or enrich the sample for the gene fusions or cells that contain the gene fusions. A variety of techniques known to those of ordinary skill in the art may be used for this purpose, including but not limited: centrifugation; immunocapture; cell lysis; and, nucleic acid target capture (See, e.g., EP Pat. No. 1 409 727).

### B. DNA and RNA Detection

The cancer markers described herein may be detected as RNA using a variety of nucleic acid techniques known to those of ordinary skill in the art, including but not limited to: nucleic acid sequencing; nucleic acid hybridization; and, nucleic acid amplification.

### 1. Sequencing

Illustrative non-limiting examples of nucleic acid sequencing techniques include, but are not limited to, chain terminator (Sanger) sequencing and dye terminator sequencing. Those of ordinary skill in the art will recognize that because RNA is less stable in the cell and more prone to nuclease attack experimentally RNA is usually reverse transcribed to DNA before sequencing.

Chain terminator sequencing uses sequence-specific termination of a DNA synthesis reaction using modified nucleotide substrates. Extension is initiated at a specific site on the template DNA by using a short radioactive, or other labeled, oligonucleotide primer complementary to the template at that region. The oligonucleotide primer is extended using a DNA polymerase, standard four deoxynucleotide bases, and a low concentration of one chain terminating nucleotide, most commonly a di-deoxynucleotide. This reaction is repeated in four separate tubes with each of the bases taking turns as the di-deoxynucleotide. Limited incorporation of the chain terminating nucleotide by the DNA polymerase results in a series of related DNA fragments that are terminated only at positions where that particular di-deoxynucleotide is used. For each reaction tube, the fragments are size-separated by electrophoresis in a slab polyacrylamide gel or a capillary tube filled with a viscous polymer. The sequence is determined by reading which lane produces a visualized mark from the labeled primer as you scan from the top of the gel to the bottom.

Dye terminator sequencing alternatively labels the terminators. Complete sequencing can be performed in a single reaction by labeling each of the di-deoxynucleotide chain-terminators with a separate fluorescent dye, which fluoresces at a different wavelength.

### 2. Hybridization

Illustrative non-limiting examples of nucleic acid hybridization techniques include, but are not limited to, *in situ* hybridization (ISH), microarray, and Southern or Northern blot.

*In situ* hybridization (ISH) is a type of hybridization that uses a labeled complementary DNA or RNA strand as a probe to localize a specific DNA or RNA sequence in a portion or section of tissue (*in situ*), or, if the tissue is small enough, the entire tissue (whole mount ISH). DNA ISH can be used to determine the structure of chromosomes. RNA ISH is used to measure and localize mRNAs and other transcripts within tissue sections or whole mounts. Sample cells and tissues are usually treated to fix the target transcripts in place and to increase access of the probe. The probe hybridizes to the target sequence at elevated temperature, and then the excess probe is washed away. The probe that was labeled with either radio-, fluorescent- or antigen-labeled bases is localized and quantitated in the tissue using either autoradiography, fluorescence microscopy or immunohistochemistry, respectively. ISH can also use two or more probes, labeled with radioactivity or the other non-radioactive labels, to simultaneously detect two or more transcripts.

### 2.1 FISH

Cancer marker sequences may be detected using fluorescence *in situ* hybridization (FISH). The preferred FISH assays utilize bacterial artificial chromosomes (BACs). These have been used extensively in the human genome sequencing project (see Nature 409: 953-958 (2001)) and clones containing specific BACs are available through distributors that can be located through many sources, e.g., NCBI. Each BAC clone from the human genome has been given a reference name that unambiguously identifies it. These names can be used to find a corresponding GenBank sequence and to order copies of the clone from a distributor.

A method of performing a FISH assay on human prostate cells, human prostate tissue or on the fluid surrounding said human prostate cells or human prostate tissue is further provided.

Specific protocols are well known in the art and can be readily adapted for use. Guidance regarding methodology may be obtained from many references including: In situ Hybridization: Medical Applications (eds. G. R. Coulton and J. de Belleroche), Kluwer Academic Publishers, Boston (1992); In situ Hybridization: In Neurobiology: Advances in Methodology (eds. J. H. Eberwine, K. L. Valentino, and J. D. Barchas), Oxford University Press Inc., England (1994); In situ Hybridization: A Practical Approach (ed. D. G. Wilkinson), Oxford University Press Inc., England (1992)); Kuo, et al., Am. J. Hum. Genet. 49:112-119 (1991); Klinger, et al., Am. J. Hum. Genet. 51:55-65 (1992); and Ward, et al., Am. J. Hum. Genet. 52:854-865 (1993)). There are also kits that are commercially available and that provide protocols for performing FISH assays (available from e.g., Oncor, Inc., Gaithersburg, MD). Patents providing guidance on methodology include U.S. Pat. Nos. 5,225,326; 5,545,524; 6,121,489 and 6,573,043. All of these references may be used along with similar references in the art and with the information provided in the Examples section herein to establish procedural steps convenient for a particular laboratory.

### 2.2 Microarrays

Different kinds of biological assays are called microarrays including, but not limited to: DNA microarrays (*e.g*., cDNA microarrays and oligonucleotide microarrays); protein microarrays; tissue microarrays; transfection or cell microarrays; chemical compound microarrays; and, antibody microarrays. A DNA microarray, commonly known as gene chip, DNA chip, or biochip, is a collection of microscopic DNA spots attached to a solid surface (*e.g*., glass, plastic or silicon chip) forming an array for the purpose of expression profiling or monitoring expression levels for thousands of genes simultaneously. The affixed DNA segments are known as probes, thousands of which can be used in a single DNA microarray. Microarrays can be used to identify disease genes by comparing gene expression in disease and normal cells. Microarrays can be fabricated using a variety of technologies, including but not limiting: printing with fine-pointed pins onto glass slides; photolithography using pre-made masks; photolithography using dynamic micromirror devices; ink-jet printing; or, electrochemistry on microelectrode arrays.

Southern and Northern blotting is used to detect specific DNA or RNA sequences, respectively. DNA or RNA extracted from a sample is fragmented, electrophoretically separated on a matrix gel, and transferred to a membrane filter. The filter bound DNA or RNA is subject to hybridization with a labeled probe complementary to the sequence of interest. Hybridized probe bound to the filter is detected. A variant of the procedure is the reverse Northern blot, in which the substrate nucleic acid that is affixed to the membrane is a collection of isolated DNA fragments and the probe is RNA extracted from a tissue and labeled.

### 3. Amplification

Cancer marker genomic DNA and mRNA may be amplified prior to or simultaneous with detection. Illustrative non-limiting examples of nucleic acid amplification techniques include, but are not limited to, polymerase chain reaction (PCR), reverse transcription polymerase chain reaction (RT-PCR), transcription-mediated amplification (TMA), ligase chain reaction (LCR), strand displacement amplification (SDA), and nucleic acid sequence based amplification (NASBA). Those of ordinary skill in the art will recognize that certain amplification techniques (*e.g.,* PCR) require that RNA be reversed transcribed to DNA prior to amplification (*e.g.,* RT-PCR), whereas other amplification techniques directly amplify RNA (*e.g.,* TMA and NASBA).

The polymerase chain reaction (U.S. Pat. Nos. 4,683,195, 4,683,202, 4,800,159 and 4,965,188), commonly referred to as PCR, uses multiple cycles of denaturation, annealing of primer pairs to opposite strands, and primer extension to exponentially increase copy numbers of a target nucleic acid sequence. In a variation called RT-PCR, reverse transcriptase (RT) is used to make a complementary DNA (cDNA) from mRNA, and the cDNA is then amplified by PCR to produce multiple copies of DNA. For other various permutations of PCR *see, e.g.,* U.S. Pat. Nos. 4,683,195, 4,683,202 and 4,800,159; Mullis et al., Meth. Enzymol. 155: 335 (1987); and, Murakawa et al., DNA 7: 287 (1988).

Transcription mediated amplification (U.S. Pat. Nos. 5,480,784 and 5,399,491), commonly referred to as TMA, synthesizes multiple copies of a target nucleic acid sequence autocatalytically under conditions of substantially constant temperature, ionic strength, and pH in which multiple RNA copies of the target sequence autocatalytically generate additional copies. *See, e.g.,* U.S. Pat. Nos. 5,399,491 and 5,824,518. In a variation described in U.S. Publ. No. 20060046265, TMA optionally incorporates the use of blocking moieties, terminating moieties, and other modifying moieties to improve TMA process sensitivity and accuracy.

The ligase chain reaction (Weiss, R., Science 254: 1292 (1991)), commonly referred to as LCR, uses two sets of complementary DNA oligonucleotides that hybridize to adjacent regions of the target nucleic acid. The DNA oligonucleotides are covalently linked by a DNA ligase in repeated cycles of thermal denaturation, hybridization and ligation to produce a detectable double-stranded ligated oligonucleotide product.

Strand displacement amplification (Walker, G. et al., Proc. Natl. Acad. Sci. USA 89: 392-396 (1992); U.S. Pat. Nos. 5,270,184 and 5,455,166), commonly referred to as SDA, uses cycles of annealing pairs of primer sequences to opposite strands of a target sequence, primer extension in the presence of a dNTPαS to produce a duplex hemiphosphorothioated primer extension product, endonuclease-mediated nicking of a hemimodified restriction endonuclease recognition site, and polymerase-mediated primer extension from the 3' end of the nick to displace an existing strand and produce a strand for the next round of primer annealing, nicking and strand displacement, resulting in geometric amplification of product. Thermophilic SDA (tSDA) uses thermophilic endonucleases and polymerases at higher temperatures in essentially the same method (EP Pat. No. 0 684 315).

Other amplification methods include, for example: nucleic acid sequence based amplification (U.S. Pat. No. 5,130,238), commonly referred to as NASBA; one that uses an RNA replicase to amplify the probe molecule itself (Lizardi et al., BioTechnol. 6: 1197 (1988), herein incorporated by reference in its entirety), commonly referred to as Qβ replicase; a transcription based amplification method (Kwoh et al., Proc. Natl. Acad. Sci. USA 86:1173 (1989)); and, self-sustained sequence replication (Guatelli et al., Proc. Natl. Acad. Sci. USA 87: 1874 (1990)). For further discussion of known amplification methods *see* Persing, David H., "In Vitro Nucleic Acid Amplification Techniques" in Diagnostic Medical Microbiology: Principles and Applications (Persing et al., Eds.), pp. 51-87 (American Society for Microbiology, Washington, DC (1993)).

### 4. Detection Methods

Non-amplified or amplified nucleic acids can be detected by any conventional means. For example, the cancer marker nucleic acid can be detected by hybridization with a detectably labeled probe and measurement of the resulting hybrids. Illustrative non-limiting examples of detection methods are described below.

One illustrative detection method, the Hybridization Protection Assay (HPA) involves hybridizing a chemiluminescent oligonucleotide probe (*e.g*., an acridinium ester-labeled (AE) probe) to the target sequence, selectively hydrolyzing the chemiluminescent label present on unhybridized probe, and measuring the chemiluminescence produced from the remaining probe in a luminometer. *See, e.g.,* U.S. Pat. No. 5,283,174 and Norman C. Nelson et al., Nonisotopic Probing, Blotting, and Sequencing, ch. 17 (Larry J. Kricka ed., 2d ed. 1995).

Another illustrative detection method provides for quantitative evaluation of the amplification process in real-time. Evaluation of an amplification process in "real-time" involves determining the amount of amplicon in the reaction mixture either continuously or periodically during the amplification reaction, and using the determined values to calculate the amount of target sequence initially present in the sample. A variety of methods for determining the amount of initial target sequence present in a sample based on real-time amplification are well known in the art. These include methods disclosed in U.S. Pat. Nos. 6,303,305 and 6,541,205. Another method for determining the quantity of target sequence initially present in a sample, but which is not based on a real-time amplification, is disclosed in U.S. Pat. No. 5,710,029.

Amplification products may be detected in real-time through the use of various self-hybridizing probes, most of which have a stem-loop structure. Such self-hybridizing probes are labeled so that they emit differently detectable signals, depending on whether the probes are in a self-hybridized state or an altered state through hybridization to a target sequence. By way of non-limiting example, "molecular torches" are a type of self-hybridizing probe that includes distinct regions of self-complementarity (referred to as "the target binding domain" and "the target closing domain") which are connected by a joining region (*e.g*., non-nucleotide linker) and which hybridize to each other under predetermined hybridization assay conditions. Molecular torches may contain single-stranded base regions in the target binding domain that are from 1 to about 20 bases in length and are accessible for hybridization to a target sequence present in an amplification reaction under strand displacement conditions. Under strand displacement conditions, hybridization of the two complementary regions, which may be fully or partially complementary, of the molecular torch is favored, except in the presence of the target sequence, which will bind to the single-stranded region present in the target binding domain and displace all or a portion of the target closing domain. The target binding domain and the target closing domain of a molecular torch include a detectable label or a pair of interacting labels (*e.g*., luminescent/quencher) positioned so that a different signal is produced when the molecular torch is self-hybridized than when the molecular torch is hybridized to the target sequence, thereby permitting detection of probe:target duplexes in a test sample in the presence of unhybridized molecular torches. Molecular torches and a variety of types of interacting label pairs are disclosed in U.S. Pat. No. 6,534,274.

Another example of a detection probe having self-complementarity is a "molecular beacon." Molecular beacons include nucleic acid molecules having a target complementary sequence, an affinity pair (or nucleic acid arms) holding the probe in a closed conformation in the absence of a target sequence present in an amplification reaction, and a label pair that interacts when the probe is in a closed conformation. Hybridization of the target sequence and the target complementary sequence separates the members of the affinity pair, thereby shifting the probe to an open conformation. The shift to the open conformation is detectable due to reduced interaction of the label pair, which may be, for example, a fluorophore and a quencher (*e.g*., DABCYL and EDANS). Molecular beacons are disclosed in U.S. Pat. Nos. 5,925,517 and 6,150,097.

Other self-hybridizing probes are well known to those of ordinary skill in the art. By way of non-limiting example, probe binding pairs having interacting labels, such as those disclosed in U.S. Pat. No. 5,928,862 might be adapted for use. Probe systems used to detect single nucleotide polymorphisms (SNPs) might also be utilized in. Additional detection systems include "molecular switches," as disclosed in U.S. Publ. No. 20050042638. Other probes, such as those comprising intercalating dyes and/or fluorochromes, are also useful for detection of amplification products. *See, e.g.,* U.S. Pat. No. 5,814,447.

### C. Protein Detection

The cancer markers may be detected as proteins using a variety of protein techniques known to those of ordinary skill in the art, including but not limited to: protein sequencing; and, immunoassays.

### 1. Sequencing

Illustrative non-limiting examples of protein sequencing techniques include, but are not limited to, mass spectrometry and Edman degradation.

Mass spectrometry can, in principle, sequence any size protein but becomes computationally more difficult as size increases. A protein is digested by an endoprotease, and the resulting solution is passed through a high pressure liquid chromatography column. At the end of this column, the solution is sprayed out of a narrow nozzle charged to a high positive potential into the mass spectrometer. The charge on the droplets causes them to fragment until only single ions remain. The peptides are then fragmented and the mass-charge ratios of the fragments measured. The mass spectrum is analyzed by computer and often compared against a database of previously sequenced proteins in order to determine the sequences of the fragments. The process is then repeated with a different digestion enzyme, and the overlaps in sequences are used to construct a sequence for the protein.

In the Edman degradation reaction, the peptide to be sequenced is adsorbed onto a solid surface (*e.g*., a glass fiber coated with polybrene). The Edman reagent, phenylisothiocyanate (PTC), is added to the adsorbed peptide, together with a mildly basic buffer solution of 12% trimethylamine, and reacts with the amine group of the N-terminal amino acid. The terminal amino acid derivative can then be selectively detached by the addition of anhydrous acid. The derivative isomerizes to give a substituted phenylthiohydantoin, which can be washed off and identified by chromatography, and the cycle can be repeated. The efficiency of each step is about 98%, which allows about 50 amino acids to be reliably determined.

### 2. Immunoassays

Illustrative non-limiting examples of immunoassays include, but are not limited to: immunoprecipitation; Western blot; ELISA; immunohistochemistry; immunocytochemistry; flow cytometry; and, immuno-PCR. Polyclonal or monoclonal antibodies detectably labeled using various techniques known to those of ordinary skill in the art (*e.g*., colorimetric, fluorescent, chemiluminescent or radioactive) are suitable for use in the immunoassays.

Immunoprecipitation is the technique of precipitating an antigen out of solution using an antibody specific to that antigen. The process can be used to identify protein complexes present in cell extracts by targeting a protein believed to be in the complex. The complexes are brought out of solution by insoluble antibody-binding proteins isolated initially from bacteria, such as Protein A and Protein G. The antibodies can also be coupled to sepharose beads that can easily be isolated out of solution. After washing, the precipitate can be analyzed using mass spectrometry, Western blotting, or any number of other methods for identifying constituents in the complex.

A Western blot, or immunoblot, is a method to detect protein in a given sample of tissue homogenate or extract. It uses gel electrophoresis to separate denatured proteins by mass. The proteins are then transferred out of the gel and onto a membrane, typically polyvinyldiflroride or nitrocellulose, where they are probed using antibodies specific to the protein of interest. As a result, researchers can examine the amount of protein in a given sample and compare levels between several groups.

An ELISA, short for Enzyme-Linked ImmunoSorbent Assay, is a biochemical technique to detect the presence of an antibody or an antigen in a sample. It utilizes a minimum of two antibodies, one of which is specific to the antigen and the other of which is coupled to an enzyme. The second antibody will cause a chromogenic or fluorogenic substrate to produce a signal. Variations of ELISA include sandwich ELISA, competitive ELISA, and ELISPOT. Because the ELISA can be performed to evaluate either the presence of antigen or the presence of antibody in a sample, it is a useful tool both for determining serum antibody concentrations and also for detecting the presence of antigen.

Immunohistochemistry and immunocytochemistry refer to the process of localizing proteins in a tissue section or cell, respectively, via the principle of antigens in tissue or cells binding to their respective antibodies. Visualization is enabled by tagging the antibody with color producing or fluorescent tags. Typical examples of color tags include, but are not limited to, horseradish peroxidase and alkaline phosphatase. Typical examples of fluorophore tags include, but are not limited to, fluorescein isothiocyanate (FITC) or phycoerythrin (PE).

Flow cytometry is a technique for counting, examining and sorting microscopic particles suspended in a stream of fluid. It allows simultaneous multiparametric analysis of the physical and/or chemical characteristics of single cells flowing through an optical/electronic detection apparatus. A beam of light (*e.g.,* a laser) of a single frequency or color is directed onto a hydrodynamically focused stream of fluid. A number of detectors are aimed at the point where the stream passes through the light beam; one in line with the light beam (Forward Scatter or FSC) and several perpendicular to it (Side Scatter (SSC) and one or more fluorescent detectors). Each suspended particle passing through the beam scatters the light in some way, and fluorescent chemicals in the particle may be excited into emitting light at a lower frequency than the light source. The combination of scattered and fluorescent light is picked up by the detectors, and by analyzing fluctuations in brightness at each detector, one for each fluorescent emission peak, it is possible to deduce various facts about the physical and chemical structure of each individual particle. FSC correlates with the cell volume and SSC correlates with the density or inner complexity of the particle (*e.g*., shape of the nucleus, the amount and type of cytoplasmic granules or the membrane roughness).

Immuno-polymerase chain reaction (IPCR) utilizes nucleic acid amplification techniques to increase signal generation in antibody-based immunoassays. Because no protein equivalence of PCR exists, that is, proteins cannot be replicated in the same manner that nucleic acid is replicated during PCR, the only way to increase detection sensitivity is by signal amplification. The target proteins are bound to antibodies which are directly or indirectly conjugated to oligonucleotides. Unbound antibodies are washed away and the remaining bound antibodies have their oligonucleotides amplified. Protein detection occurs via detection of amplified oligonucleotides using standard nucleic acid detection methods, including real-time methods.

### D. Data Analysis

A computer-based analysis program may be used to translate the raw data generated by the detection assay (*e.g*., the presence, absence, or amount of a given marker or markers) into data of predictive value for a clinician. The clinician can access the predictive data using any suitable means. Thus, the clinician, who is not likely to be trained in genetics or molecular biology, need not understand the raw data. The data is presented directly to the clinician in its most useful form. The clinician is then able to immediately utilize the information in order to optimize the care of the subject.

Any method capable of receiving, processing, and transmitting the information to and from laboratories conducting the assays, information provides, medical personal, and subjects may be utilized. For example, a sample (*e.g.,* a biopsy or a serum or urine sample) may be obtained from a subject and submitted to a profiling service (*e.g*., clinical lab at a medical facility, genomic profiling business, etc.), located in any part of the world (*e.g*., in a country different than the country where the subject resides or where the information is ultimately used) to generate raw data. Where the sample comprises a tissue or other biological sample, the subject may visit a medical center to have the sample obtained and sent to the profiling center, or subjects may collect the sample themselves (*e.g*., a urine sample) and directly send it to a profiling center. Where the sample comprises previously determined biological information, the information may be directly sent to the profiling service by the subject (*e.g*., an information card containing the information may be scanned by a computer and the data transmitted to a computer of the profiling center using an electronic communication systems). Once received by the profiling service, the sample is processed and a profile is produced (*i.e.*, expression data), specific for the diagnostic or prognostic information desired for the subject.

The profile data is then prepared in a format suitable for interpretation by a treating clinician. For example, rather than providing raw expression data, the prepared format may represent a diagnosis or risk assessment (e.g., likelihood of cancer being present or the subtype of cancer) for the subject, along with recommendations for particular treatment options. The data may be displayed to the clinician by any suitable method. For example, the profiling service generates a report that can be printed for the clinician (*e.g*., at the point of care) or displayed to the clinician on a computer monitor.

The information may first be analyzed at the point of care or at a regional facility. The raw data is then sent to a central processing facility for further analysis and/or to convert the raw data to information useful for a clinician or patient. The central processing facility provides the advantage of privacy (all data is stored in a central facility with uniform security protocols), speed, and uniformity of data analysis. The central processing facility can then control the fate of the data following treatment of the subject. For example, using an electronic communication system, the central facility can provide data to the clinician, the subject, or researchers.

The subject may be able to directly access the data using the electronic communication system. The subject may chose further intervention or counseling based on the results. The data may be used for research use. For example, the data may be used to further optimize the inclusion or elimination of markers as useful indicators of a particular condition or stage of disease.

The results may be used in a clinical setting to determine a further diagnostic (e.g., the additional of further screening (e.g., PSA or other markers) or diagnostic (e.g., biopsy) course of action. Alternatively, the results are used to determine a treatment course of action (e.g., choice of therapies or watchful waiting).

### E. In vivo Imaging

*In vivo* imaging techniques are used to visualize the expression of cancer markers in an animal (*e.g*., a human or non-human mammal). For example, cancer marker mRNA or protein may be labeled using a labeled antibody specific for the cancer marker. A specifically bound and labeled antibody can be detected in an individual using an *in vivo* imaging method, including, but not limited to, radionuclide imaging, positron emission tomography, computerized axial tomography, X-ray or magnetic resonance imaging method, fluorescence detection, and chemiluminescent detection. Methods for generating antibodies to the cancer markers of are described above.

*In vivo* imaging methods are useful in the diagnosis of cancers that express the cancer markers described herein (*e.g.,* SPINK1). *In vivo* imaging is used to visualize the presence of a marker indicative of the cancer. Such techniques allow for diagnosis without the use of an unpleasant biopsy. The *in vivo* imaging methods are also useful for providing prognoses to cancer patients. For example, the presence of a marker indicative of cancers likely to metastasize can be detected. The *in vivo* imaging methods can further be used to detect metastatic cancers in other parts of the body.

Reagents (*e.g*., antibodies) specific for the cancer markers may be fluorescently labeled. The labeled antibodies are introduced into a subject (*e.g*., orally or parenterally). Fluorescently labeled antibodies are detected using any suitable method *(e.g*., using the apparatus described in U.S. Patent 6,198,107).

Antibodies may be radioactively labeled. The use of antibodies for *in vivo* diagnosis is well known in the art. Sumerdon et al., (Nucl. Med. Biol 17:247-254 [1990] have described an optimized antibody-chelator for the radioimmunoscintographic imaging of tumors using Indium-111 as the label. Griffin et al., (J Clin One 9:631-640 [1991]) have described the use of this agent in detecting tumors in patients suspected of having recurrent colorectal cancer. The use of similar agents with paramagnetic ions as labels for magnetic resonance imaging is known in the art (Lauffer, Magnetic Resonance in Medicine 22:339-342 [1991]). The label used will depend on the imaging modality chosen. Radioactive labels such as Indium-111, Technetium-99m, or Iodine-131 can be used for planar scans or single photon emission computed tomography (SPECT). Positron emitting labels such as Fluorine-19 can also be used for positron emission tomography (PET). For MRI, paramagnetic ions such as Gadolinium (III) or Manganese (II) can be used.

Radioactive metals with half-lives ranging from 1 hour to 3.5 days are available for conjugation to antibodies, such as scandium-47 (3.5 days) gallium-67 (2.8 days), gallium-68 (68 minutes), technetiium-99m (6 hours), and indium-111 (3.2 days), of which gallium-67, technetium-99m, and indium-111 are preferable for gamma camera imaging, gallium-68 is preferable for positron emission tomography.

A useful method of labeling antibodies with such radiometals is by means of a bifunctional chelating agent, such as diethylenetriaminepentaacetic acid (DTPA), as described, for example, by Khaw et al. (Science 209:295 [1980]) for In-111 and Tc-99m, and by Scheinberg et al. (Science 215:1511 [1982]). Other chelating agents may also be used, but the 1-(p-carboxymethoxybenzyl)EDTA and the carboxycarbonic anhydride of DTPA are advantageous because their use permits conjugation without affecting the antibody's immunoreactivity substantially.

Another method for coupling DPTA to proteins is by use of the cyclic anhydride of DTPA, as described by Hnatowich et al. (Int. J. Appl. Radiat. Isot. 33:327 [1982]) for labeling of albumin with In-111, but which can be adapted for labeling of antibodies. A suitable method of labeling antibodies with Tc-99m which does not use chelation with DPTA is the pretinning method of Crockford et al., (U.S. Pat. No. 4,323,546, herein incorporated by reference).

A preferred method of labeling immunoglobulins with Tc-99m is that described by Wong et al. (Int. J. Appl. Radiat. Isot., 29:251 [1978]) for plasma protein, and recently applied successfully by Wong et al. (J. Nucl. Med., 23:229 [1981]) for labeling antibodies.

In the case of the radiometals conjugated to the specific antibody, it is likewise desirable to introduce as high a proportion of the radiolabel as possible into the antibody molecule without destroying its immunospecificity.

A further improvement may be achieved by effecting radiolabeling in the presence of the specific cancer marker, to insure that the antigen binding site on the antibody will be protected. The antigen is separated after labeling.

*In vivo* biophotonic imaging (Xenogen, Almeda, CA) may be utilized for *in vivo* imaging. This real-time *in vivo* imaging utilizes luciferase. The luciferase gene is incorporated into cells, microorganisms, and animals (*e.g*., as a fusion protein with a cancer marker). When active, it leads to a reaction that emits light. A CCD camera and software is used to capture the image and analyze it.

### F. Compositions and Kits

In yet other embodiments, kits for the detection and characterization of prostate cancer are provided. In some embodiments, the kits contain antibodies specific for a cancer marker (e.g., SPINK1), in addition to detection reagents and buffers. In other embodiments, the kits contain reagents specific for the detection of the level of mRNA or cDNA or the presence or absence of chromosomal deletions (*e.g*., oligonucleotide probes or primers). In preferred embodiments, the kits contain all of the components necessary or sufficient to perform a detection assay, including all controls, directions for performing assays, and any necessary software for analysis and presentation of results.

### IV. Drug Screening

Drug screening assays (*e.g.,* to screen for anticancer drugs) are provided. The screening methods utilize cancer markers identified herein (*e.g*., including but not limited to, SPINK1). For example, methods of screening for compounds that alter (*e.g*., decrease or increase) the expression of cancer marker genes or alter the health of individuals expression, not expressing, or possessing a particular cancer marker are disclosed. Candidate compounds are antisense agents (*e.g.*, oligonucleotides) directed against cancer markers. See Section IV below for a discussion of antisense therapy. Candidate compounds are also antibodies or small molecules that specifically bind to a cancer marker and inhibit its biological function.

In one screening method, candidate compounds are evaluated for their ability to alter cancer marker expression by contacting a compound with a cell expressing a cancer marker and then assaying for the effect of the candidate compounds on expression. The effect of candidate compounds on expression of a cancer marker gene may be assayed for by detecting the level of cancer marker mRNA expressed by the cell. mRNA expression can be detected by any suitable method. The effect of candidate compounds on expression of cancer marker genes may be assayed by measuring the level of polypeptide encoded by the cancer markers. The level of polypeptide expressed can be measured using any suitable method, including but not limited to, those disclosed herein.

Specifically, screening methods for identifying modulators, *i.e.,* candidate or test compounds or agents (*e.g*., proteins, peptides, peptidomimetics, peptoids, small molecules or other drugs) which bind to cancer markers described herein, have an inhibitory (or stimulatory) effect on, for example, cancer marker expression or cancer marker activity, or have a stimulatory or inhibitory effect on, for example, the expression or activity of a cancer marker substrate are provided. Compounds thus identified can be used to modulate the activity of target gene products (*e.g*., cancer marker genes) either directly or indirectly in a therapeutic protocol, to elaborate the biological function of the target gene product, or to identify compounds that disrupt normal target gene interactions. Compounds that inhibit the activity or expression of cancer markers are useful in the treatment of proliferative disorders, *e.g*., cancer, particularly prostate cancer.

Assays for screening candidate or test compounds that are substrates of a cancer marker protein or polypeptide or a biologically active portion thereof are disclosed. Assays for screening candidate or test compounds that bind to or modulate the activity of a cancer marker protein or polypeptide or a biologically active portion thereof are also disclosed.

The test compounds can be obtained using any of the numerous approaches in combinatorial library methods known in the art, including biological libraries; peptoid libraries (libraries of molecules having the functionalities of peptides, but with a novel, non-peptide backbone, which are resistant to enzymatic degradation but which nevertheless remain bioactive; see, *e.g.,* Zuckennann et al., J. Med. Chem. 37: 2678-85 [1994]); spatially addressable parallel solid phase or solution phase libraries; synthetic library methods requiring deconvolution; the 'one-bead one-compound' library method; and synthetic library methods using affinity chromatography selection. The biological library and peptoid library approaches are preferred for use with peptide libraries, while the other four approaches are applicable to peptide, non-peptide oligomer or small molecule libraries of compounds (Lam (1997) Anticancer Drug Des. 12:145).

Examples of methods for the synthesis of molecular libraries can be found in the art, for example in: DeWitt et al., Proc. Natl. Acad. Sci. U.S.A. 90:6909 [1993]; Erb et al., Proc. Nad. Acad. Sci. USA 91:11422 [1994]; Zuckermann et al., J. Med. Chem. 37:2678 [1994]; Cho et al., Science 261:1303 [1993]; Carrell et al., Angew. Chem. Int. Ed. Engl. 33.2059 [1994]; Carell et al., Angew. Chem. Int. Ed. Engl. 33:2061 [1994]; and Gallop et al., J. Med. Chem. 37:1233 [1994].

Libraries of compounds may be presented in solution (*e.g*., Houghten, Biotechniques 13:412-421 [1992]), or on beads (Lam, Nature 354:82-84 [1991]), chips (Fodor, Nature 364:555-556 [1993]), bacteria or spores (U.S. Patent No. 5,223,409), plasmids (Cull et al., Proc. Nad. Acad. Sci. USA 89:18651869 [1992]) or on phage (Scott and Smith, Science 249:386-390 [1990]; Devlin Science 249:404-406 [1990]; Cwirla et al., Proc. NatI. Acad. Sci. 87:6378-6382 [1990]; Felici, J. Mol. Biol. 222:301 [1991]).

### V. Cancer Therapies

Therapies for cancer (*e.g*., prostate cancer) are disclosed. Therapies may target cancer markers (*e.g*., including but not limited to, SPINK1). For example, antisense or RNAi therapies that target the expression of SPINK1 may be utilized. Additional therapeutic agents are identified, for example, using the drug screening methods described herein.

### EXPERIMENTAL

The following examples are provided in order to demonstrate and further illustrate certain preferred embodiments and aspects and are not to be construed as limiting the scope thereof.

### Example 1: Outlier Expression of SPINK1 in Prostate Cancer

This Example describes the characterization of SPINK1 expression in prostate cancer.

### A. Materials and Methods

### Cancer Outlier Profile Analysis (COPA)

COPA analysis was performed on 7 prostate cancer gene expression data sets (Yu et al., J Clin Oncol 22, 2790-9 (2004); Lapointe et al., Proc Natl Acad Sci USA 101, 811-6 (2004); Glinsky et al., J Clin Invest 113, 913-23 (2004); Vanaja et al., Cancer Res 63, 3877-82 (2003), Dhanasekaran et al., Nature 412, 822-6 (2001), LaTulippe et al., Cancer Res 62, 4499-506 (2002); Welsh et al., Cancer Res 61, 5974-8 (2001)) in Oncomine 3.0 as described (Tomlins et al. Science 310, 644-8 (2005)). COPA has three steps. First, gene expression values are median-centered, setting each gene's median expression value to zero. Second, the median absolute deviation (MAD) is calculated and scaled to 1 by dividing each gene expression value by its MAD. Of note, median and MAD were used for transformation as opposed to mean and standard deviation so that outlier expression values do not unduly influence the distribution estimates, and are thus preserved post normalization. Third, the 75th, 90th, and 95th percentiles of the transformed expression values are tabulated for each gene and then genes are rank-ordered by their percentile scores, providing a prioritized list of outlier profiles. Genes scoring in the top 100 outliers at any of the three percentile cutoffs were called outliers. Genes identified as outliers in the same number of studies were further ranked by their average outlier rank in those outlier studies. SPINK1 expression was also interrogated in prostate cancer specimens from two multi-cancer profiling studies (Bittner et al., Nat Biotechnol 23, 183-4 (2005), Su et al., Cancer Res 61, 7388-93 (2001)).

### Samples

Tissues used for quantitative PCR were from the radical prostatectomy series at the University of Michigan and from the Rapid Autopsy Program, which are both part of University of Michigan Prostate Cancer Specialized Program of Research Excellence (S.P.O.R.E.) Tissue Core. For combined fluorescence *in situ* hybridization (FISH) and immunohistochemistry (IHC) evaluation, the University of Michigan (UM) cohort consisted of samples from the radical prostatectomy series. The Swedish Watchful Waiting (SWW) cohort consisted of samples from a Swedish population-based cohort of men with localized prostate cancer diagnosed incidentally by trans-urethral resection of the prostate for symptomatic benign prostatic hyperplasia as described (Andren et al., J Urol 175, 1337-40 (2006); Johansson et al., JAMA 291, 2713-9 (2004)). The Memorial Sloan Kettering Cancer Center (MSKCC) cohort consisted of patients with localized or locally advanced prostate cancer that were treated by radical prostatectomy at MSKCC between 1985 and 2003. All samples were obtained with IRB approval from the respective institution. The prostate cancer cell line 22RV1 was provided by Jill Macoska (University of Michigan).

### Quantitative PCR (qPCR) from tissue samples

Quantitative PCR (qPCR) was performed using SYBR Green dye on an Applied Biosystems 7300 Real Time PCR system (Applied Biosystems, Foster City, CA) essentially as described (Tomlins et al., Science 310, 644-8 (2005); Tomlins et al., Cancer Res 66, 3396-400 (2006)). Briefly, total RNA was isolated from tissues using Trizol (Invitrogen, Carlsbad, CA). RNA was quantified using a ND-1000 spectrophotometer (Nanodrop Technologies, Wilmington, DE) and 3-5 µg of total RNA was reverse transcribed into cDNA using SuperScript III (Invitrogen) in the presence of random primers. All qPCR reactions were performed with Power SYBR Green Master Mix (Applied Biosystems) and 25 ng of both the forward and reverse primer using the manufacturer's recommended thermocycling conditions. For each experiment, threshold levels were set during the exponential phase of the qPCR reaction using Sequence Detection Software version 1.2.2 (Applied Biosystems). The amount of ERG, ETV1 and SPINK1 relative to the average of the house keeping genes GAPDH and HMBS for each sample was determined using the comparative threshold cycle (Cₜ) method (according to the Applied Biosystems User Bulletin #2). The relative amount of ERG, ETV1 and SPINK1 for each sample was calibrated to the median amount from all samples for each gene. All oligonucleotide primers were synthesized by Integrated DNA Technologies (Coralville, IA). GAPDH and HMBS, and ERG (exon5_6) and ETV1 (exon6_7) primers were as described (Tomlins et al., 2005, supra). Sequences for SPINK1 are as follows:
SPINK1_f- CAAAAATCTGGGCCTTGCTGAGAAC (SEQ ID NO:1)
SPINK1_r- AGGCCTCGCGGTGACCTGAT (SEQ ID NO:2)

Approximately equal efficiencies of the primers were confirmed using serial dilutions of pooled prostate cancer cDNA in order to use the comparative Cₜ method. All reactions were subjected to melt curve analysis.

### Immunohistochemistry (IHC) and fluorescence in situ hybridization (FISH)

IHC for the University of Michigan (UM) and Swedish Watchful Waiting (SWW) cohorts was performed using a mouse monoclonal antibody against SPINK1 (H00006690-M01, Abnova, Taipei City, Taiwan) on tissue microarrays containing cores from 75 (UM) and 312 (SWW) evaluable cases of localized prostate cancer. Staining in greater than 1% of cancerous epithelial cells was deemed positive. Previously, we have evaluated cases on these tissue microarrays for TMRPSS2:ERG fusion status by FISH using break apart ERG assays as previously described (Tomlins et al., 2005, supra). A one-sided Fisher's exact test was used to evaluate the relationship between SPINK1 and fusion status, as these studies were performed with the prior hypothesis that there was an inverse correlation between SPINK1 expression and fusion status.

### MSKCC Immunohistochemistry

IHC for the MSKCC cohort was performed using an in-house mouse monoclonal antibody against SPINK1 (code 6E832) on tissue microarrays containing triplicate cores from 817 evaluable cases of localized prostate cancer. The percentage of positive tumor cells in each core was estimated and assigned values of 0%, 5%, or multiples of 10%. The intensity of the expression was assigned a value of 0, 1, 2, or 3. Triplicate cores from each specimen were scored separately and the presence of tumorous tissue in at least two interpretable cores was required to include a case for analysis. Cases were marked as SPINK1 positive if any of the three cores showed >8% of cancerous cells showing positive SPINK1 immunoreactivity (intensity 1-3).

### Outcome Analyses

For Kaplan-Meier analysis of the Glinsky et al. and UM datasets, biochemical recurrence was defined as a 0.2 ng/ml increase in PSA or recurrence of disease after prostatectomy, such as development of metastatic cancer, if biochemical recurrence information was not available. For the MSKCC cohort, only biochemical recurrence, defined as PSA > 0.2 ng/ml after surgical resection with a second confirmatory PSA-measurement > 0.2 ng/ml, was considered, as all patients with a clinical failure had previously had a biochemical recurrence. For outcome analysis from the Glinsky et al. dataset, samples positive for outlier expression of SPINK1 were defined as those with greater than 0.5 normalized expression units (as shown in Figure 1A). For the IHC analysis of the UM and MSKCC cohorts, positive cases were defined as described above. Kaplan-Meier analysis and multivariate Cox proportional-hazards regression were then used to examine the association of SPINK1 with biochemical PSA recurrence. To predict the probability of disease recurrence, the Kattan 7-year post-operative nomogram (Kattan et al., J Clin Oncol 17, 1499-507 (1999)) was used, and the concordance index of the nomogram and the nomogram plus SPINK1 status was evaluated using 1000 times bootstrapping as described (Kattan et al., J Clin Oncol 21, 3573-9 (2003)).

### Urine based detection of SPINK1 expression

The collection of urine, isolation of RNA, RNA amplification and qPCR for TMRPSS2:ERG from men with prostate cancer was as described (Laxman, B. et al. Noninvasive detection of TMPRSS2:ERG fusion transcripts in the urine of men with prostate cancer. Neoplasia 8, 885-8 (2006)). Briefly, 25 ng of isolated RNA was amplified using TransPlex Whole Transcriptome Amplification (WTA) kit (Rubicon Genomics, Ann Arbor, MI) according to the manufacturer's instructions. For each qPCR reaction, 10 ng of WTA amplified cDNA was used as template. 2x Power SYBR Green Master Mix (Applied Biosystems, Foster City, CA) and 25 ng of both the forward and reverse primers were used for SPINK1, ERG (primers as described above) and PSA (Laxman et al., 2006, supra). For all experiments, the same threshold and baseline was set using Sequence Detection Software version 1.2.2 (Applied Biosystems). All samples with a threshold cycle (Cₜ) value greater than 26 for PSA were excluded to remove samples with insufficient prostate cell recovery. Samples were considered TMRPSS2:ERG positive if both ERG and TMRPSS2-ERG assays showed Cₜ values less than 37. The amount of SPINK1 relative to PSA was determined for each sample using the comparative Cₜ method and the relative amount in each sample was normalized to the median of all samples. The top 11% of SPINK1 over-expressing samples (the average percentage of SPINK1 positive samples across the other five cohorts evaluated in this study (See Table 3)) were identified as SPINK1 positive. A one-sided Fisher's exact test was used to evaluate the relationship between SPINK1 and TMPRSS2:ERG status, as this study was performed with the prior hypothesis that there was an inverse correlation between SPINK1 expression and fusion status.

### In vitro over-expression of SPINK1

cDNA of SPINK1 (NM_003122.2), as present in a clinical prostate cancer specimen over-expressing SPINK1, was amplified by RT-PCR using the following primers, with the forward primer including a consensus Kozak sequence (start and stop codons underlined):
SPINK1_full-f: ACCACCATGAAGGTAACAGGCATCTTTCTT (SEQ ID NO:3)
SPINK1_full-r: TCAGCAAGGCCCAGATTTTTGA (SEQ ID NO:4)
The cDNA product was TOPO cloned into the Gateway entry vector pCR8/GW/TOPO (Invitrogen), yielding pCR8-SPINK1. To generate adenoviral constructs, pCR8-SPINK1 was recombined with pAD/CMV/V5 (Invitrogen) using LR Clonase II (Invitrogen). Control pAD/CMV/LACZ clones were obtained from Invitrogen. Adenoviruses were generated by the University of Michigan Vector Core. The benign immortalized prostate cell line RWPE was infected with SPINK1 or LACZ adenoviruses, generating RWPE-SPINK1 and RWPE-LACZ for transient over-expression.

### Proliferation assay

Proliferation for RWPE-LACZ and RWPE-SPINK1 cells was measured by a colorimetric assay based on the cleavage of the tetrazolium salt WST-1 by mitochondrial dehydrogenases (cell proliferation reagent WST1, Roche Diagnostics, Mannheim, Germany) at the indicated time points in triplicate. Cell counts for 22RV1 cells were estimated by trypsinizing cells and analysis by Coulter counter (Beckman Coulter, Fullerton, CA) at 72 hours in triplicate.

### Invasion assays

For invasion assays, RWPE-SPINK1 and RWPE-LACZ cells (48 hours after infection with adenoviruses), or 22RV1 cells were used. Equal numbers of the indicated cells were seeded onto the basement membrane matrix (EC matrix, Chemicon, Temecula, CA) present in the insert of a 24 well culture plate, with fetal bovine serum added to the lower chamber as a chemoattractant. After 48 hours, non-invading cells and EC matrix were removed by a cotton swab. Invaded cells were stained with crystal violet and photographed. The inserts were treated with 10% acetic acid and absorbance was measured at 560nm.

### SPINK1 knockdown

For siRNA knockdown of SPINK1 in 22RV1 cells, the individual siRNAs composing the Dharmacon SMARTpool against SPINK1 (LQ-019724-00, Chicago, IL) were tested for SPINK1 knockdown by qPCR, and the most effective single siRNA (J-019724-07) was used for further experiments. siCONTROL Non-Targeting siRNA #1 (D-001210-01) or siRNA against SPINK1 was transfected into 22RV1 cells using Oligofectamine (Invitrogen). After 24 hours a second identical transfection was carried out and cells were harvested 24 hours later for RNA isolation, invasion assays or proliferation assays as described above.

### Expression Profiling

Expression profiling was performed using the Agilent Whole Human Genome Oligo Microarray (Santa Clara, CA). Total RNA isolated using Trizol was purified using the Qiagen RNAeasy Micro kit (Valencia, CA). One µg of total RNA was converted to cRNA and labeled according to the manufacturer's protocol (Agilent). Hybridizations were performed for 16 hrs at 65°C, and arrays were scanned on an Agilent DNA microarray scanner. Images were analyzed and data extracted using Agilent Feature Extraction Software 9.1.3.1, with linear and lowess normalization performed for each array. For 22RV1-siSPINK1 hybridizations, the reference was 22RV1 cells infected with non-targeting siRNA. Duplicate hybridizations were performed with duplicate dye flips, for a total of four arrays. Over and under-expressed signatures were generated by filtering to include only features with significant differential expression (PValueLogRatio < 0.01) in all hybridizations and Cy5/Cy3 ratios > or < 1 in all hybridizations.

### B. Results

As described above, SPINK1 (serine peptidase inhibitor, Kazal type 1), the 2nd ranked meta-outlier, was identified as showing over-expression in prostate cancer compared to benign prostate tissue and mutually exclusive over-expression with ERG and ETV1 across multiple studies. The profile of SPINK1 expression and scatter plots with ERG and ETV1 for two studies (Glinsky et al., J Clin Invest 113, 913-23 (2004); Yu et al., J Clin Oncol 22, 2790-9 (2004)) where SPINK1 was identified as a top 100 outlier are shown in Figure 1b, with plots from 7 additional studies (Dhanasekaran et al., Nature 412, 822-6 (2001); LaTulippe et al., Cancer Res 62, 4499-506 (2002); Vanaja et al., Cancer Res 63, 3877-82 (2003); Welsh et al., Cancer Res 61, 5974-8 (2001); Su et al., Cancer Res 61, 7388-93 (2001), GSE2109 and GSE8218) measuring SPINK1 expression shown in Figure 5. In total, from these studies, SPINK1 showed outlier expression in only 2 of 127 (1.6%) benign prostate tissue samples and 64 of 387 (16.5%) prostate cancers (two sided Fisher's exact test, p = 9.5E-7). Three hundred eighty four of 387 profiled prostate cancers (99.2%) showed mutually exclusive over-expression of SPINK1, ERG, and ETV11, as shown in Figures 1b and 5.

To confirm the outlier expression of SPINK1 exclusively in ETS negative prostate cancers, SPINK1, ERG and ETV1 expression was measured by quantitative PCR (qPCR) in an independent cohort of 10 benign prostate tissues and 61 prostate cancers (54 clinically localized and 7 metastatic samples). While ERG, ETV1, or SPINK1 was markedly over-expressed in 25 (41%), 4 (6.5%), and 4 (6.5%) of 61 prostate cancers, respectively, no benign prostate tissue samples demonstrated over-expression of these genes. Confirming the profiling studies described above, ERG, ETV1 and SPINK1 were over-expressed in distinct samples (Fig 6).

After demonstrating that outlier expression of SPINK1 defines a subset of ETS rearrangement negative prostate cancers at the transcript level, the expression of SPINK1 protein in prostate cancers was evaluated. By immunohistochemical (IHC) analysis on tissue microarrays, SPINK1 expression was evaluated in two independent cohorts, (University of Michigan (UM) and Swedish Watchful Waiting (SWW)) representing a total of 392 cases of clinically localized prostate cancers. Both cohorts have been previously evaluated for TMRPSS2:ERG fusion status by fluorescence *in situ* hybridization (FISH). In both cohorts, prostate cancer epithelia exhibited either strong or no expression of SPINK1, without intermediate staining as observed for many prostate cancer markers. As shown in Figures 2a-b, in the UM cohort, 10 and 36 of 75 cases were positive for SPINK1 expression (13.3%) and TMRPSS2:ERG fusions (48%), respectively, with all SPINK1 positive cases being TMRPSS2:ERG negative (one sided Fisher's exact test, p = 0.0008). In the SWW cohort, 23 and 57 of 312 cases were positive for SPINK1 expression (7.4%) and TMRPSS2:ERG fusions (18.3%), respectively, again with all SPINK1 positive cases being TMRPSS2:ERG negative (one sided Fisher's exact test, p = 0.008).

Approximately 25-40% of patients treated by radical prostatectomy for clinically localized prostate cancer will experience disease recurrence, initially indicated by an increase in the serum level of PSA (biochemical recurrence) (Han et al., Urol Clin North Am 28, 555-65 (2001); Hull et al., G.W. et al. Cancer control with radical prostatectomy alone in 1,000 consecutive patients. J Urol 167, 528-34 (2002)). Thus, it was next determined if SPINK1 outlier status was associated with biochemical recurrence after surgical resection. Two datasets were identified from the evaluated cohorts for which there was follow-up biochemical recurrence information and a sufficient number of SPINK1 positive cases (>5). The Glinsky gene expression dataset, which contained tumors from 79 patients (with 37 recurrences), 10 of which showed outlier mRNA transcript expression of SPINK1 (>0.5 normalized expression units), was examined. These patients had a significantly higher risk of recurrence than patients without outlier SPINK1 expression (hazard ratio: 2.65, 95% CI: 1.16-6.07, log rank p = 0.016) by Kaplan-Meier analysis (Figure 2c). Multivariate Cox proportional-hazards regression analysis also revealed that SPINK1 outlier status, independent of Gleason score, lymph node status, surgical margin status, age and pre-operative PSA, was a significant predictor of clinical recurrence of prostate cancer (hazard ratio: 2.5; 95% CI: 1.1-6.0; p = 0.035, Table 2).

The same analysis was next performed on the UM cohort (75 cases, 28 recurrences) evaluated for SPINK1 status by IHC. By Kaplan-Meier analysis, SPINK1 positive staining was significantly associated with biochemical recurrence (hazard ratio: 2.49, 95% CI: 1.01-6.18, p = 0.04, Figure 2d). Multivariate Cox proportional-hazards regression analysis again confirmed that SPINK1 status predicted recurrence independently of other clinical parameters (Table 2). With an adjusted hazard ratio of 4.1 (95% CI: 1.4-11.7, p = 0.009), it was the strongest predictor in this model.

As a final validation, IHC for SPINK1 status was performed on an independent cohort of 817 evaluable prostate cancers (200 recurrences) from the Memorial Sloan Kettering Cancer Center (MSKCC). In this MSKCC cohort, with IHC performed independently from the UM and SWW cohorts using a different SPINK1 antibody, 297 of the 817 cases (36%) of cases showed positive SPINK1 immunoreactivity in at least one of three triplicate cores. In addition, staining intensity was more variable than that observed in the UM and SWW cohorts. As the percentage of cases in this cohort with SPINK1 staining (36%) is far greater than the other IHC cohorts (13% and 7%) or the percentage of SPINK1 outlier samples from DNA microarray and qPCR studies (17% and 7%, see Table 3), SPINK1 positive cases in the MSKCC cohort were defined as those with at least one core showing greater than 80% of cells showing positive SPINK1 immunoreactivity, resulting in 75 SPINK1 positive cases (9%), consistent with the other studies. By Kaplan-Meier analysis, SPINK1 positive cases in the MSKCC cohort showed significantly shorter time to biochemical recurrence (hazard ratio: 2.32, 95% CI: 1.59-3.39, P = 6.96E-06, Figure 2e). Multivariate Cox proportional-hazards regression analysis again confirmed that SPINK1 outlier status, independent of Gleason score, lymph node status, surgical margin status, seminal vesicle invasion, extracapsular extension and pre-operative PSA, was a significant predictor of clinical recurrence (hazard ratio: 2.02; 95% CI: 1.37-2.99; p = 0.0004, Table 2). Clinically, nomograms are commonly used to predict the likelihood of biochemical recurrence after surgical resection by optimally incorporating clinical and pathological parameters. To determine whether the addition of SPINK1 improves a validated nomogram for predicting the 7-year post-prostatectomy probability of biochemical recurrence (Kattan et al., J Clin Oncol 17, 1499-507 (1999)), the concordance index (Kattan et al., J Clin Oncol 21, 3573-9 (2003)) (the probability that given two randomly selected patients, the patient with the worse outcome is indeed predicted to have a worse outcome) of the nomogram and the nomogram plus SPINK1 status were assessed. The bootstrap-corrected concordance index was minimally improved in all three datasets by the addition of SPINK1 status to the nomogram (Glinsky et al: 0.772 vs 0.762, UM IHC: 0.698 vs 0.676 and MSKCC: 0.775 vs. 0.765). Thus, while SPINK1 does not dramatically add to the predictive ability of an optimized multivariate model, it was demonstrated by analyzing 971 cancers from three cohorts that SPINK1 outlier status identifies an aggressive subset of prostate cancers.

It was next determined if outlier expression of SPINK1 could be detected non-invasively. As serum levels of SPINK1 may be dysregulated as the result of a number of malignancies (Paju and Stenman, Crit Rev Clin Lab Sci 43, 103-42 (2006); Stenman et al., Clin Chem 48, 1206-9 (2002) and 44% of patients with prostate cancer are reported to have elevated serum levels of SPINK1 (Paju et al., Eur Urol (2007)), a specific assay to identify the ~11% of patients with outlier expression (See Table 3) was developed. The detection of TMRPSS2:ERG fusion transcripts in the urine of men with prostate cancer was recently described (Laxman et al., Neoplasia 8, 885-8 (2006)), and this assay allows one to more directly assess transcripts contributed by prostatic cells. Thus SPINK1 expression was assessed from a cohort of 148 urine samples collected from men with prostate cancer that have been characterized as TMRPSS2:ERG positive (43) or negative (105). As shown in Figure 2f, while 1 of the 43 TMRPSS2:ERG positive samples (2.3%) showed marked SPINK1 over-expression (top 11% of normalized SPINK1 expression), 15 of the 105 TMRPSS2:ERG negative samples (14%) showed marked SPINK1 over-expression (Fisher's exact test, p = 0.02).

To investigate a functional role for SPINK1 in prostate cancer, adenoviruses expressing SPINK1 were generated and benign immortalized prostate epithelial cell line RWPE was infected with the adenovirus to generate RWPE-SPINK1 cells. Over-expression of SPINK1 had no significant effect on the proliferation or invasion of RWPE cells (Figure 3a-b). As SPINK1 over-expression had no effect on benign prostate cells, we hypothesized that SPINK1 over-expression may occur later in prostate cancer progression in the presence of co-existing genetic lesions, consistent with its association with aggressive prostate cancer. Thus, a panel of prostate cancer cell lines was analyzed for SPINK1 outlier-expression. Outlier expression of SPINK1 was identified exclusively in the 22RV1 cell line (Figure 3c), consistent with previous work (Paju et al., supra). The aggressive 22RV1 prostate cancer cell line was derived from a human prostate carcinoma xenograft that was serially propagated in nude mice after castration-induced regression and relapse of the parental, androgen-dependent CWR22 xenograft (Sramkoski, R.M. et al. A new human prostate carcinoma cell line, 22Rv1. In Vitro Cell Dev Biol Anim 35, 403-9 (1999)). 22RV1 does not over-express ERG or ETV1 (Figure 3c), similar to clinical SPINK1 outlier cases, supporting its use as a cell line model of SPINK1 outlier expression.

To assess the function of SPINK1 in 22RV1, siRNA knockdown was utilized. While SPINK1 knockdown had no affect on 22RV1 proliferation (Figure 3d), SPINK1 knockdown markedly attenuated the invasiveness of 22RV1 cells through a modified basement membrane (Figures 3e-f). Consistent with the mutually exclusive over-expression of ERG, ETV1 and SPINK1, siRNA knockdown of ERG or ETV1 in 22RV1 had no effect on invasion, while SPINK1 knockdown had no effect on the invasiveness of VCaP (TMPRSS2:ERG positive) or LNCaP (ETV1 rearrangement positive) (Figures 3g-h). siRNA knockdown of ERG in VCaP and ETV1 in LNCaP similarly attenuated invasion (Figures 3g-h) without affecting proliferation (Tomlins et al., 2007, supra). Additionally, microarray analysis of 22RV1-siSPINK1 cells revealed only limited transcriptional effects (76 features over-expressed, 14 features under-expressed, Table 4 and Figure 7), indicating that SPINK1 knockdown directly affects cellular invasiveness. Together, these results provide a role for SPINK1 in prostate cancer invasion, consistent with its over-expression in aggressive prostate cancers.

The outlier expression of SPINK1 in a subset of prostate cancers suggests that SPINK1 may be activated by a unique molecular event, similar to TMPRSS2:ETS positive prostate cancers. However, FISH studies using locus/control and 5'/3' split probes demonstrated no evidence of amplification or gross rearrangements, respectively, in samples with SPINK1 over-expression. Additionally, sequencing of the SPINK1 coding region identified no mutations in samples with SPINK1 outlier-expression. The present disclosure is not limited to a particular mechanism. Indeed, an understanding of the mechanism is not necessary to understand the invention. Nonetheless, it is contemplated that SPINK1 is activated by increased transcription, for example through promoter mutations affecting regulatory elements. Alternatively, SPINK1 may be activated by a unique upstream genetic event. However, few genes show consistent correlation with SPINK1 across data sets (Fig 8), indicating that SPINK1 is an exclusive downstream target. It is also possible that SPINK1 is downregulated in TMPRSS2:ETS positive cancers, however one would expect high SPINK1 expression in benign prostatic epithelium and the *in vitro* data described above supports a role for SPINK1 over-expression in prostate cancer progression.

In conclusion, using a combination of *in silico* bioinformatics analysis coupled with independent experimental validation, data on 1,800 prostate cancers was analyzed, demonstrating the consistent outlier expression of SPINK1 in TMPRSS2:ETS negative prostate cancers (Table 3). Evidence is provided that SPINK1 over-expression defines an aggressive molecular sub-type of prostate cancer (~11% of cases) not attributable to known gene fusion events. It was demonstrated that SPINK1 can be monitored non-invasively in urine and thus serves to complement gene fusion based urine testing for prostate cancer. Additionally, the utility of 22RV1 as a cell line model for SPINK1 outlier expression was demonstrated. In further experiments, a meta-COPA strategy was used to nominate candidate oncogenes in specific cancer types.

**Table 1**

| Meta-COPA analysis of 7 prostate cancer gene expression profiling datasets in Oncomine. Genes were ranked by the number of studies in which they scored in the top 100 outliers (ranked by COPA) at any of the three pre-defined percentile cutoffs (75th, 90th, 95th). Genes were further ranked by their average COPA rank (Avg. Rank) in studies where they ranked in the top 100. | | | |
|---|---|---|---|
| **Meta COPA Rank** | **Gene** | **# of Studies** | **Avg. Rank** |
| 1 | *ERG* | 7 | 19.3 |
| 2 | *SPINK1* | 5 | 29.8 |
| 3 | *GPR116* | 5 | 46 |
| 4 | *ORM1* | 4 | 10 |
| 5 | *ETV1* | 4 | 23 |
| 6 | *MVL2* | 4 | 26.8 |
| 7 | *NEB* | 4 | 27 |
| 8 | *TGM4* | 4 | 30.8 |
| 9 | *NELL2* | 4 | 33.5 |
| 10 | *KRT13* | 4 | 49 |
| 11 | *SLC26A4* | 4 | 63.3 |
| 12 | *MYL1* | 3 | 8.7 |
| 13 | *CXCL13* | 3 | 11 |
| 14 | *HCG3* | 3 | 12 |
| 15 | *HPGD* | 3 | 20.7 |
| 16 | *MIPEP* | 3 | 27.3 |
| 17 | *PLAT* | 3 | 31.7 |
| 18 | *CST1* | 3 | 32.3 |
| 19 | *COL2A1* | 3 | 35.3 |
| 20 | *CPS1* | 3 | 36.3 |
| 21 | *CRISP3* | 3 | 36.7 |
| 22 | *CTAG1A* | 3 | 42.3 |
| 23 | *FGB* | 3 | 42.3 |
| 24 | *PPFIA2* | 3 | 42.7 |
| 25 | *CDH2* | 3 | 44 |
| 26 | *PTPRM* | 3 | 45.3 |
| 27 | *CYP3A43* | 3 | 51.7 |
| 28 | *PROM1* | 3 | 53 |
| 29 | *HDAC9* | 3 | 61.7 |

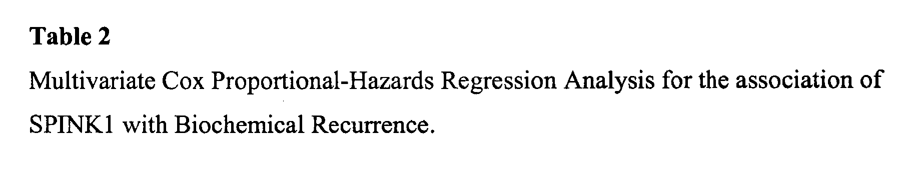

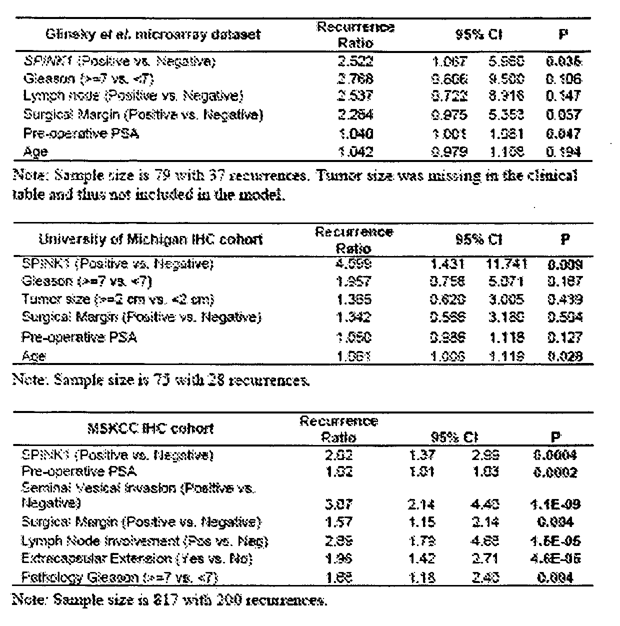

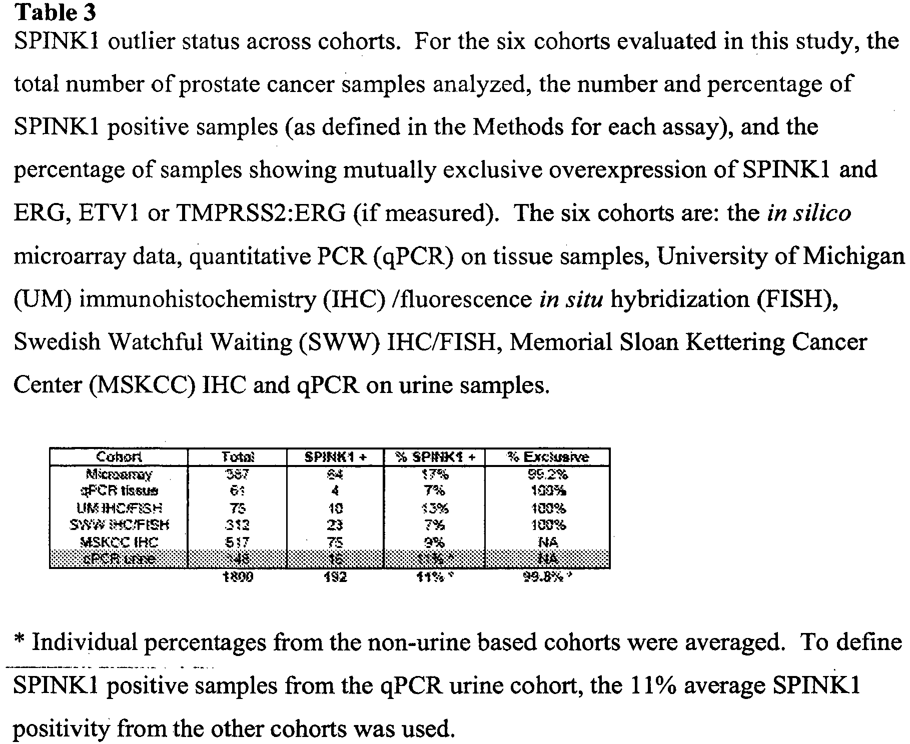

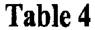

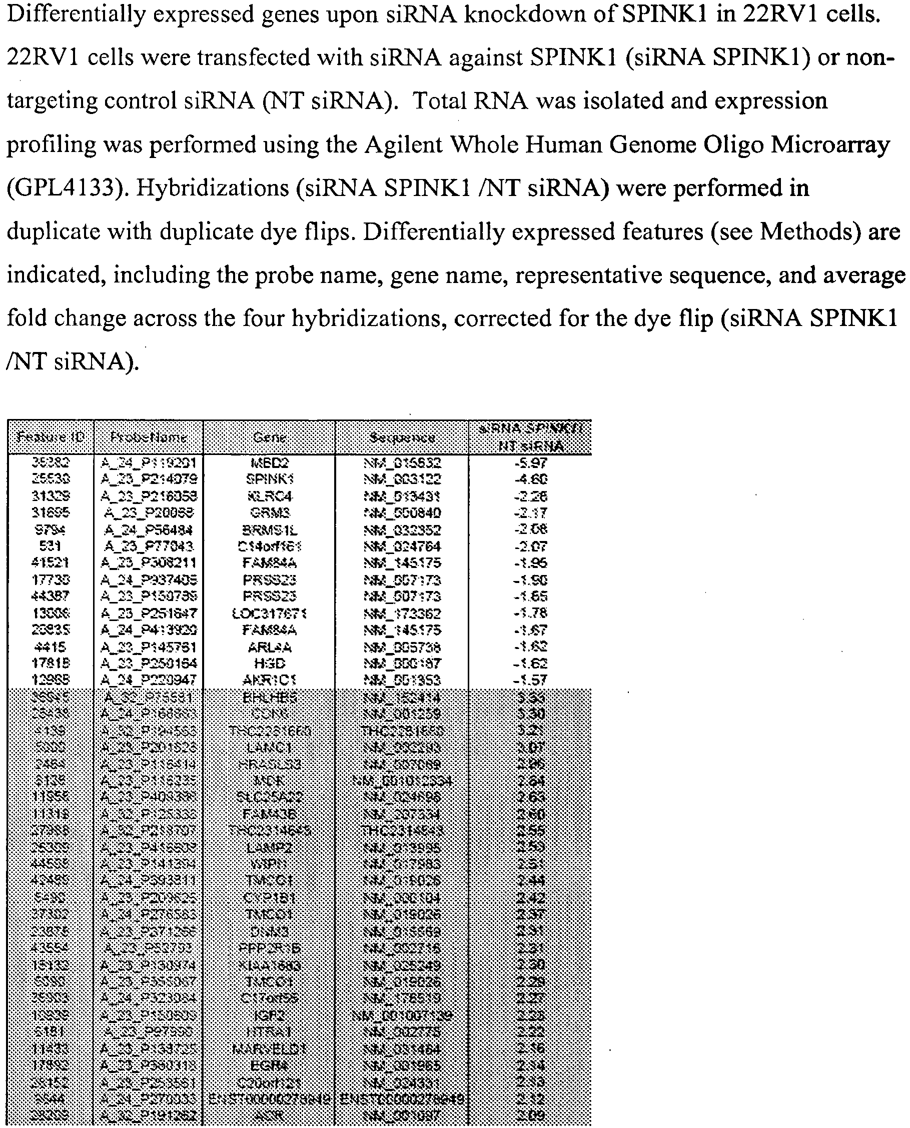

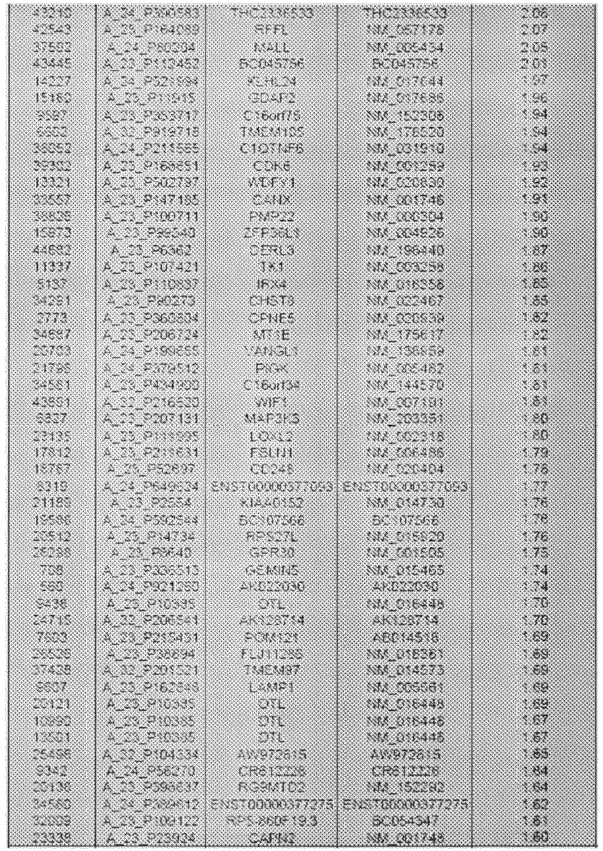

### Example 2: Multiplex Urine Assay

This example describes a multiplex urine assay for use in the assessment of samples for markers associated with prostate cancer.

### A. Methods

### Urine Collection, RNA isolation, amplification and quantitative PCR

This study was approved by the Institutional Review Board (IRB) of the University of Michigan Medical School and samples were obtained from 276 patients with informed consent following a digital rectal exam before either needle biopsy (n=216) or radical prostatectomy (n=60) at the University of Michigan Health System (UMHS). Urine was voided into urine collection cups containing DNA/RNA preservative (Sierra Diagnostics LLC, Sonora, CA). Isolation of RNA from urine and whole transcriptome amplification (WTA) were as described in (Laxman et al., Neoplasia 2006;8:885-8.). Quantitative PCR (qPCR) was used to detect seven prostate cancer biomarkers (AMACR, ERG, GOLPH2, PCA3, SPINK1, TFF3, and TMPRSS2:ERG fusions) and the control transcripts PSA and GAPDH from WTA amplified cDNA essentially as described (Laxman et al., 2006, supra; Tomlins et al., Neoplasia 2006;8:153-62). The primer sequences for ERG (exon5_6) (Tomlins et al., Science 2005;310:644-8), GAPDH (Vandesompele et al., Genome Biol 2002; 3:RESEARCH0034), AMACR (Kumar-Sinha et al., Am J Pathol 2004;164:787-93), and PSA (Specht et al., Am J Pathol 2001;158:419-29) were previously described and for other biomarkers were as follows:
GOLPH2-f: CTGGTGGCCTGCATCATCGTCTTG (SEQ ID NO:5),
GOLPH2-r: GCTGCTCCCGCTGCTTCTCCA (SEQ ID NO:6),
PCA3-f: CATGGTGGGAAGGACCTGATGATAC (SEQ ID NO:7),
PCA3-r: GATGTGTGGCCTCAGATGGTAAAGTC (SEQ ID NO:8),
SPINK1-f: CAAAAATCTGGGCCTTGCTGAGAAC (SEQ ID NO:9),
SPINK1-r: AGGCCTCGCGGTGACCTGAT (SEQ ID NO:10),
TFF3-f: AACCGGGGCTGCTGCTTTGACTC (SEQ ID NO:11),
TFF3-r: TCCTGCAGGGGCTTGAAACACCA (SEQ ID NO:12).
TMPRSS2:ERG fusions were detected using Taqman primers/probe, with the following
sequences:
TM-ERGa3-f: CTGGAGCGCGGCAGGAA (SEQ ID NO: 13),
TM-ERGa3 -r: CCGTAGGCACACTCAAACAACGA (SEQ ID NO: 14),
TM-ERGa3_MGB-probe: 5'-MGB-TTATCAGTTGTGAGTGAGGAC-3' (SEQ ID NO: 15).
Threshold levels were set during the exponential phase of the qPCR reaction using Sequence Detection Software version 1.2.2 (Applied Biosystems, Foster City, CA), with the same baseline and threshold set for each plate, to generate threshold cycle (Cₜ) values for all genes for each sample.

### Analysis

qPCR was performed on WTA cDNA from urine collected from 111 biopsy-negative patients and 165 patients with prostate cancer (105 biopsy positive patients and 60 prostatectomy patients). Samples that had PSA Cₜ values greater than 27 were excluded to ensure sufficiency of the amount of prostate cells in the samples, leading to 105 biopsy-negative and 152 samples from patients with prostate cancer in the analysis. For qPCR analysis, raw -ΔCₜ was used (to stabilize the variance of testing variables) as opposed to testing markers against control (2^{-ΔCt}). TMPRSS2:ERG was dichotomized as a binary variable to reflect the fusion positive or negative status observed in tissue samples (Tomlins et al., 2005, supra, Perner et al., Am J Surg Pathol 2007;31:882-8), with positive samples defined as those with Cₜ values less than 37. As PCA3 has been reported to be a prostate tissue-specific marker (de Kok et al., Cancer Res 2002;62:2695-8), it was normalized against urine PSA (C_{tPSA}-C_{tPCA3}). All other testing variables were adjusted against their mean urine PSA and GAPDH values ((C_{tPSA}+C_{tGAPDH})/ 2-C_{tVariable}). Twenty two samples showing outlier values were excluded, as at least one testing variable in those samples showed an adjusted value below 3 standard deviations from its sample mean, indicating qPCR failure. This resulted in a final data set of samples from 138 patients with prostate cancer (86 positive needle biopsy and 52 radical prostatectomy patients) and 96 biopsy-negative patients.

### Statistical analysis

Univariate and multivariate logistic regressions were used to examine associations between prostate cancer diagnostic status and testing variables. For multivariate logistic regression, the Akaike Information Criterion (AIC)-based backward selection was used to drop insignificant terms (Venables WNaR, B. D Modern Applied Statistics with S, 4th edition.: New York: Springer, 2002). All testing markers were included in the initial regression model which was further refined by the AIC-based backward selection. After the final model was determined, the predicted probability for each sample was used as input to generate the receiver operating characteristic (ROC) curve and the area under the curve (AUC) was calculated. As all samples were used for regression model generation, the estimated AUC may be over-optimized. To correct this bias, a leave-one-out cross validation was performed. Briefly, one sample was omitted while the regression model was trained on the remaining samples to select optimal markers and estimate their coefficients. The prediction probability is then calculated based on the model prediction for the left-out sample. This was repeated until every sample was left out once and the generated prediction probability values were then used for ROC analysis. Similarly, PCA3 was fitted in a logistic regression model to generate an AUC. The difference of AUCs was examined as described previously (DeLong et al., Biometrics 1988;44:837-45). All analyses were performed in R and ROC curves were plotted in SPSS 11.5 (SPSS Inc., Chicago, IL, USA).

### Risk Stratification

Clinical information was identified from the medical record to determine association with clinical factors such as clinical stage, Gleason score, and risk categories based on biopsy results and pathologic data (D'Amico et al., JAMA 1998;280:969-74). Clinical nomograms were used to calculate risk of progression free survival and pathologic staging (Kattan et al., J Natl Cancer Inst 1998;90:766-71; Kattan et al., Cancer 1997;79:528-37). A novel risk stratification grouping (High risk vs. low risk) was also evaluated by grouping all Gleason 6 tumors, any Gleason 7 tumors with clinical T1c stage or Gleason 3 +4 tumors as a low risk tumors and Gleason 8+, stage T2 Gleason 7 tumors or tumors with Gleason 4 +3 grading as high risk tumors. All variables were tested for univariate association with each clinical risk group.

### B. Results

To develop a multiplexed qPCR based test for prostate cancer, we assessed seven putative prostate cancer biomarkers based on previously published reports and analysis from our group in a final cohort of 138 patients with prostate cancer (86 positive needle biopsy and 52 radical prostatectomy patients) and 96 patients with negative needle biopsies. Biomarkers included those generally over-expressed in prostate cancer, such as PCA3, AMACR and GOLPH2 (Rubin et al., JAMA 2002;287:1662-70; de Kok et al., supra), as well as those over-expressed in subsets of prostate cancers, such as ERG and TMPRSS2:ERG, and TFF3 and SPINK1 (Tomlins et al, 2005, supra; Faith et al., Prostate 2004;61:215-27; Garraway et al., Prostate 2004;61:209-14).

All genes were first tested by univariate analysis, with GOLPH2 (P = 0.0002), SPINK1 (P = 0.0002), PCA3 (P = 0.001) and TMPRSS2:ERG fusion (P = 0.034) showing significant association for discriminating patients with prostate cancer from patients with negative needle biopsies (Figure 9 and Table 5). Both AMACR, which has previously been shown to be a sensitive and specific biomarker for prostate cancer in tissues (Rubin et al., 2002, supra) and TFF3, which shows high expression in a subset of prostate cancers (Faith et al., Prostate 2004;61:215-27; Garraway et al., Prostate 2004;61:209-14), were not statistically significant predictors of prostate cancer using urine samples (P = 0.450 and 0.189, respectively). The lack of specificity of AMACR and TFF3 in urine may be due to expression of these transcripts in urothelial or kidney derived cellular material which can also be shed in the urine. While TMPRSS2:ERG fusion was significantly associated with the presence of prostate cancer (Figure 9 and Table 5), ERG overexpression was not associated with cancer presence on univariate analysis (P = 0.166), indicating that cells from other tissues may be contributing ERG transcripts to the urine. Additionally, serum PSA levels prior to biopsy or prostatectomy were also not associated with cancer presence in this cohort (P = 0.376). When tested as individual variables for the ability to detect prostate cancer based on the receiver-operating-characteristic curves (ROC), GOLPH2 (area under the curve (AUC) = 0.664, P = 2.01E-5), PCA3 (AUC = 0.661, P =2.84E-5), and SPINK1 (AUC = 0.642, P = 0.0002) outperformed serum PSA (AUC=0.508, p=0.837) (Figure 9). Thus, this study identified a number of novel biomarkers for the non-invasive detection of prostate cancer using patient urine instead of biopsy samples.

To determine if a multiplex model could improve on the performance of these single biomarkers, the analyzed prostate cancer biomarkers were next tested in a multivariate regression analysis using Akaike Information Criterion (AIC)-based backward selection (Venables, B. D Modern Applied Statistics with S, 4th edition.: New York: Springer, 2002) to drop insignificant terms from the model. This analysis resulted in a final model that included SPINK1 (P = 7.41E-5), PCA3 (P = 0.003), GOLPH2 (P = 0.004) and TMPRSS2:ERG (P = 0.006) (Table 5). To evaluate the performance of this model for diagnosing prostate cancer, ROC analysis was performed based on the predicted probabilities derived from the final model. The ROC curves from the multiplexed model and PCA3 alone were compared, as urine based detection of PCA3 has previously been evaluated in similar cohorts as a single biomarker using alternative detection technologies (Venables WNaR, B. D Modern Applied Statistics with S, 4th edition.: New York: Springer, 2002; Hessels et al., Eur Urol 2003;44:8-15; discussion -6; Fradet et al., Urology 2004;64:311-5; discussion 5-6; Groskopf et al., Clin Chem 2006;52:1089-95; Marks et al., Urology 2007;69:532-5). For example, van Gils et al. demonstrated that in a cohort of 534 men presenting for prostate biopsy with serum PSA between 3-15 ng/mL, urinary PCA3 detection expression had an AUC of 0.66 compared to 0.57 for serum PSA (van Gils et al., 2007, supra). As shown in Figure 10A, in the cohort described herein, the AUC for the multiplexed model (0.758, P = 1.91E-11) was significantly improved (P=0.003 (DeLong et al., Biometrics 1988;44:837-45)) compared to the AUC for PCA3 alone (0.662, P = 2.58E-5). At the point on the multiplex model ROC with the maximum sum of sensitivity and specificity (65.9% and 76.0%, respectively), the positive predictive value was 79.8% and the negative predictive value was 60.8% (Figure 10A). It was demonstrated that PCA3 showed improved AUC compared to serum PSA. It was also demonstrated that a multiplex model including PCA3 significantly improves the predictive ability of PCA3 alone, demonstrating the ability to improve PCA3 and other single-gene based diagnostic tests.

As all samples were used to select the best subset of variables for regression analysis, this has the potential to over-optimize the reported AUC. Thus, a leave one-out-cross validation (LOOCV) strategy was used to generate an unbiased AUC. As shown in Figure 10B, the AUC for the LOOCV multiplex model (0.736) is again significantly better (P = 0.006) than that for LOOCV PCA3 alone (0.645). At the point on the LOOCV multiplex model ROC with the maximum sum of sensitivity and specificity (62.3% and 75.0%, respectively), the positive predictive value was 78.2% and the negative predictive value was 58.1% (Figure 10B).

The ability of these genetic markers to predict clinical risk groups based on patient parameters was next assayed. Clinical risk groups were determined by clinical patient data that direct the decision to pursue biopsy, to determine treatment, or to stratify patients for surveillance regimens (see Methods). Only limited association between these prostate cancer biomarkers and clinical risk groups, with GOLPH2, SPINK1 and
TMPRSS2:ERG status showing association with risk groups was observed.
Similar to the previously described PCR based test for breast cancer recurrence risk, a prostate cancer risk test can be used to drive high risk patients to therapies more suited for their disease course (van Gils et al., supra).

In summary, this example describes a multiplexed qPCR based assay on sedimented urine collected from patients presenting for prostate biopsy or prostatectomy that exhibits superior performance relative to serum PSA or PCA3 alone. The multiplex urine test, which is a combination of PCA3, SPINK1, GOLPH2 and TMPRSS2:ERG status achieves a specificity and positive predictive value of >75%, making it a useful test to complement serum PSA, which has poor specificity in detecting prostate cancer.

**Table 5**

| **Univariate Logistic Regression Analysis** | | |
|---|---|---|
| **Variable** | **Coefficient** | **P-value** |
| *GOLPH22* | 0.4444 | 0.0002 |
| *SPINK1* | 0.25 | 0.0002 |
| *PCA3* | 0.187 | 0.001 |
| *TMPRSS2:ERG* | 0.609 | 0.034 |
| *ERG* | 0.043 | 0.166 |
| *TFF3* | 0.11 | 0.189 |
| PSA (serum) | 0.0151 | 0.376 |
| *AMACR* | 0.049 | 0.45 |
| | | |

| **Multivariate Logistic Regression Analysis** | | |
|---|---|---|
| **Variable** | **Coefficient** | **P-value** |
| *SPINK1* | 0.308 | 7.41 E-05 |
| *PCA3* | 0.191 | 0.003 |
| *GOLPH2* | 0.372 | 0.004 |
| *TMPRSS2:ERG* | 0.924 | 0.006 |

## Claims

1. A method for identifying prostate cancer in a patient comprising:
(a) detecting the expression level of SPINK1 in a patient sample containing prostate cells or a prostatic secretion from the patient and comparing the detected SPINK1 expression level with normal expression of SPINK1; and
(b) detecting the expression level of ERG and/or ETV1, if present, in the sample,
wherein detecting overexpression of SPINK1 and detecting normal expression of ERG and/or ETV1 identifies prostate cancer in the patient.

2. A method according to claim 1, comprising detecting overexpression of SPINK1 in the sample compared to normal expression of SPINK1 and detecting normal expression of ERG and ETV1 in the sample;
wherein detecting overexpression of SPINK1 and detecting normal expression of ERG and ETV1 identifies prostate cancer in the patient.

3. The method of claim 1 or claim 2, wherein step (b) comprises detecting SPINK1 RNA.

4. The method of claim 1 or claim 2, wherein step (b) comprises detecting SPINK1 protein.

5. The method of claim 1 or claim 2, wherein the sample comprises prostate tissue, blood, urine, semen, prostatic secretions or isolated prostate cells.

6. The method of claim 1 or claim 2, comprising detecting overexpression of SPINK1 mRNA or SPINK1 protein in the sample, and thereby identifying an aggressive prostate cancer in the patient.

7. The method of claim 1 or claim 2, wherein detecting overexpression of SPINK1 identifies an increased risk of recurrence of prostate cancer in the patient following radical prostatectomy.

8. A kit comprising at least one of the following:
(a) a first labelled oligonucleotide probe comprising a sequence that hybridizes specifically to SPINK1 RNA or cDNA, a second labelled oligonucleotide probe comprising a sequence that hybridizes specifically to ERG RNA or cDNA, and a third labelled oligonucleotide probe comprising a sequence that hybridizes specifically to ETV 1 RNA or cDNA; r
(b) a first pair of amplification oligonucleotides wherein each amplification oligonucleotide in the first pair comprises a sequence that hybridizes specifically to SPINK1 RNA or cDNA, in addition to (i) a second pair of amplification oligonucleotides wherein each amplification oligonucleotide in the second pair comprises a sequence that hybridizes specifically to ERG RNA or cDNA, and/or (ii) a third pair of amplification oligonucleotides wherein each amplification oligonucleotide comprises a sequence that hybridizes specifically to ETV1 RNA or cDNA; or
(c) a first antibody that binds specifically to SPINK1 protein, in addition to (i) a second antibody that binds specifically to ERG protein, and/or (ii) a third antibody that binds specifically to ETV1 protein.

## Patentansprüche

1. Verfahren zur Identifikation von Prostatakrebs in einem Patienten, das Folgendes umfasst:
(a) Detektieren der Expressionsstärke von SPINK1 in einer Patientenprobe, die Prostatazellen oder ein Prostatasekret vom Patienten enthält, und Vergleichen der detektierten Expressionsstärke von SPINK1 mit der normalen Expression von SPINK1; und
(b) Detektieren der Expressionsstärke von ERG und/oder ETV1, falls vorhanden, in der Probe, wobei das Detektieren einer Überexpression von SPINK1 und das Detektieren von normaler Expression von ERG und/oder ETV1 Prostatakrebs im Patienten identifiziert.

2. Verfahren nach Anspruch 1, welches das Detektieren von Überexpression von SPINK1 in der Probe im Vergleich zu normaler Expression von SPINK1 und das Detektieren von normaler Expression von ERG und ETV1 in der Probe umfasst;
wobei das Detektieren einer Überexpression von SPINK1 und das Detektieren von normaler Expression von ERG und ETV1 Prostatakrebs im Patienten identifiziert.

3. Verfahren nach Anspruch 1 oder Anspruch 2, wobei Schritt (b) das Detektieren von SPINK1-RNA umfasst.

4. Verfahren nach Anspruch 1 oder Anspruch 2, wobei Schritt (b) das Detektieren von SPINK1-Protein umfasst.

5. Verfahren nach Anspruch 1 oder Anspruch 2, wobei die Probe Prostatagewebe, Blut, Urin, Sperma, Prostatasekrete oder isolierte Prostatazellen umfasst.

6. Verfahren nach Anspruch 1 oder Anspruch 2, welches das Detektieren einer Überexpression von SPINK1-mRNA oder SPINK1-Protein in der Probe umfasst, wodurch ein aggressiver Prostatakrebs im Patienten identifiziert wird.

7. Verfahren nach Anspruch 1 oder Anspruch 2, wobei das Detektieren einer Überexpression von SPINK1 ein erhöhtes Risiko für das Wiederauftreten von Prostatakrebs im Patienten nach einer radikalen Prostatektomie identifiziert.

8. Set, das zumindest eines aus Folgendem umfasst:
(a) eine erste markierte Oligonucleotidsonde, die eine Sequenz umfasst, die spezifisch an SPINK1-RNA oder -cDNA hybridisiert, und eine zweite markierte Oligonucleotidsonde, die eine Sequenz umfasst, die spezifisch an ERG-RNA oder -cDNA hybridisiert, und eine dritte markierte Oligonucleotidsonde, die eine Sequenz umfasst, die spezifisch an ETV1-RNA oder -cDNA hybridisiert;
(b) ein erstes Paar von Amplifikationsoligonucleotiden, wobei jedes Amplifikationsoligonucleotid im ersten Paar eine Sequenz umfasst, die spezifisch an SPINK1-RNA oder -cDNA hybridisiert, zusätzlich zu (i) einem zweiten Paar von Amplifikationsoligonucleotiden, wobei jedes Amplifikationsoligonucleotid im zweiten Paar eine Sequenz umfasst, die spezifisch an ERG-RNA oder -cDNA hybridisiert, und/oder (ii) einem dritten Paar von Amplifikationsoligonucleotiden, wobei jedes Amplifikationsoligonucleotid eine Sequenz umfasst, die spezifisch an ETV1-RNA oder -cDNA hybridisiert; oder
(c) einen ersten Antikörper, der spezifisch an ein SPINK1-Protein bindet, zusätzlich zu (i) einem zweiten Antikörper, der spezifisch an ein ERG-Protein bindet, und/oder (ii) einem dritten Antikörper, der spezifisch an ein ETV1-Protein bindet.

## Revendications

1. Procédé pour identifier le cancer de la prostate chez un patient, comprenant :
(a) la détection du taux d'expression de SPINK1 dans un échantillon du patient contenant des cellules de la prostate ou une sécrétion prostatique du patient et la comparaison du taux d'expression de SPINK1 détecté avec l'expression normale de SPINK1 ; et
(b) la détection du taux d'expression d'ERG et/ou d'ETV1, si présent, dans l'échantillon, où la détection d'une surexpression de SPINK1 et la détection d'une expression normale d'ERG et/ou d'ETV1 identifie le cancer de la prostate chez le patient.

2. Procédé selon la revendication 1, comprenant la détection d'une surexpression de SPINK1 dans l'échantillon par comparaison à l'expression normale de SPINK1 et la détection d'une expression normale d'ERG et d'ETV1 dans l'échantillon ;
où la détection d'une surexpression de SPINK1 et la détection d'une expression normale d'ERG et d'ETV1 identifie le cancer de la prostate chez le patient.

3. Procédé selon la revendication 1 ou la revendication 2, dans lequel l'étape (b) comprend la détection de l'ARN de SPINK1.

4. Procédé selon la revendication 1 ou la revendication 2, dans lequel l'étape (b) comprend la détection de la protéine SPINK1.

5. Procédé selon la revendication 1 ou la revendication 2, dans lequel l'échantillon comprend un tissu de la prostate, du sang, de l'urine, du sperme, des sécrétions prostatiques ou des cellules de la prostate isolées.

6. Procédé selon la revendication 1 ou la revendication 2, comprenant la détection d'une surexpression de l'ARNm de SPINK1 ou de la protéine SPINK1 dans l'échantillon, en identifiant ainsi un cancer de la prostate agressif chez le patient.

7. Procédé selon la revendication 1 ou la revendication 2, dans lequel la détection d'une surexpression de SPINK1 identifie un risque accru de récidive du cancer du la prostate chez le patient suite à une prostatectomie radicale.

8. Kit comprenant au moins un des éléments suivants :
(a) une première sonde oligonucléotidique marquée comprenant une séquence qui s'hybride spécifiquement à l'ARN ou à l'ADNc de SPINK1, une deuxième sonde oligonucléotidique marquée comprenant une séquence qui s'hybride spécifiquement à l'ARN ou à l'ADNc d'ERG, et une troisième sonde oligonucléotidique marquée comprenant une séquence qui s'hybride spécifiquement à l'ARN ou à l'ADNc d'ETV1 ;
(b) une première paire d'oligonucléotides d'amplification, où chaque oligonucléotide d'amplification dans la première paire comprend une séquence qui s'hybride spécifiquement à l'ARN ou à l'ADNc de SPINK1, en plus de (i) une deuxième paire d'oligonucléotides d'amplification, où chaque oligonucléotide d'amplification dans la deuxième paire comprend une séquence qui s'hybride spécifiquement à l'ARN ou à l'ADNc d'ERG, et/ou (ii) une troisième paire d'oligonucléotides d'amplification, où chaque oligonucléotide d'amplification comprend une séquence qui s'hybride spécifiquement à l'ARN ou à l'ADNc d'ETV1 ; ou
(c) un premier anticorps qui se lie spécifiquement à la protéine SPINK1, en plus de (i) un deuxième anticorps qui se lie spécifiquement à la protéine ERG, et/ou (ii) un troisième anticorps qui se lie spécifiquement à la protéine ETV1.
